(19) Europäisches Patentamt
European Patent Office
Office européen des brevets

(11) **EP 4 692 080 A1**

(12) **EUROPEAN PATENT APPLICATION**
published in accordance with Art. 153(4) EPC

(43) Date of publication:
**11.02.2026 Bulletin 2026/07**

(21) Application number: **24777924.2**

(22) Date of filing: **22.03.2024**

(51) International Patent Classification (IPC):
***C07D 413/14*** *(2006.01)* ***A61K 31/4545*** *(2006.01)*
***A61K 31/496*** *(2006.01)* ***A61P 35/00*** *(2006.01)*

(52) Cooperative Patent Classification (CPC):
**A61K 31/4545; A61K 31/496; A61P 35/00; C07D 413/14**

(86) International application number:
**PCT/CN2024/083346**

(87) International publication number:
**WO 2024/199135 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.03.2023 CN 202310294950**

(71) Applicant: **Zhangzhou Pien Tze Huang Pharmaceutical Co., Ltd.**
**Fujian Province, 363000 (CN)**

(72) Inventors:
- **WANG, Liju**
  **Zhangzhou, Fujian 363000 (CN)**
- **LIN, Danwei**
  **Zhangzhou, Fujian 363000 (CN)**
- **JIAN, Kaili**
  **Zhangzhou, Fujian 363000 (CN)**
- **YANG, Xiaoping**
  **Zhangzhou, Fujian 363000 (CN)**
- **ZHANG, Li**
  **Zhangzhou, Fujian 363000 (CN)**
- **LUO, Yunfu**
  **Shanghai 200131 (CN)**
- **XU, Yu**
  **Shanghai 200131 (CN)**

(74) Representative: **Dehns**
**10 Old Bailey**
**London EC4M 7NG (GB)**

(54) **PREPARATION METHOD FOR SALT FORM AND CRYSTAL FORM OF 2,6-PIPERIDINEDIONE COMPOUND AND USE THEREOF**

(57) Disclosed are a preparation method for a salt form and a crystal form of a 2,6-piperidinedione compound represented by formula (I) and a use thereof.

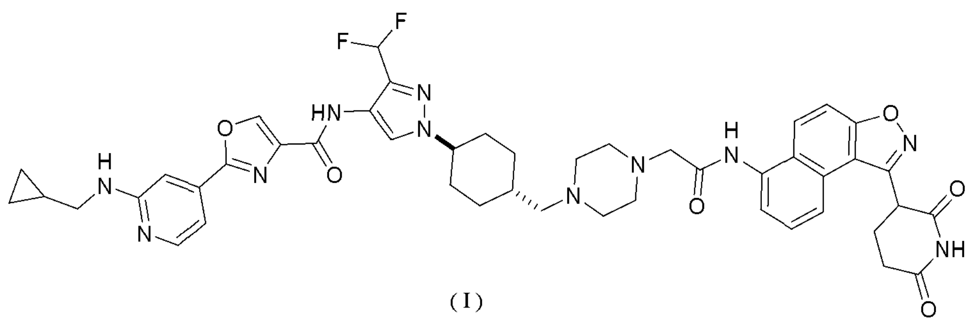

(I)

EP 4 692 080 A1

## Description

The present application claims the following priority:

The present application claims the priority and right of Chinese Patent Application No. 2023102949509 filed with the China National Intellectual Property Administration on March 24, 2023. The contents of the Chinese patent application are incorporated herein by reference in its entirety.

TECHNICAL FIELD

The present disclosure relates to a preparation method for a salt form and a crystal form of a 2,6-piperidinedione compound and a use thereof.

BACKGROUND

Interleukin-1 receptor-associated kinase 4 (IRAK4) plays a pivotal role in the signal transduction pathways of the Toll-like receptor (TLR) family and the interleukin-1 receptor (IL-1R) family, receiving upstream signals to activate downstream JNK and NF-κB signaling pathways. IRAK4 is closely associated with the development and progression of human inflammatory immune diseases and tumors.

The Toll-like receptor (TLR) signaling transduction protein myeloid differentiation factor (MyD88) is frequently mutated in various lymphomas, such as Waldenström macroglobulinemia, lymphoplasmacytic lymphoma, immune-resistant large B-cell lymphoma, and marginal zone lymphoma, with mutation rates of 95-97%, 79%, 50-80%, 15-29%, and 6-10%, respectively. IRAK4 is involved in nearly all biological functions of MyD88, making it a highly attractive and promising drug target, particularly for the treatment of MyD88-driven lymphomas.

Research has found that IRAK4 not only phosphorylates proteins but also forms complexes with MyD88 to exert its biological functions. The activation of the JNK signaling pathway by IRAK4 requires its phosphorylation function, whereas the activation of the NF-κB signaling pathway does not, indicating that IRAK4 possesses both protein kinase and scaffolding protein functions and plays a role in signaling pathways. Therefore, conventional small molecule kinase inhibitors targeting IRAK4 cannot completely block all biological functions of IRAK4.

Proteolysis targeting chimera (PROTAC) is a technique that applies the ubiquitin-proteasome system to target specific proteins and induce their intracellular degradation. The ubiquitin-proteasome system is the main pathway for intracellular protein degradation, mainly responsible for the removal of denatured, mutated, or harmful proteins from the cell as its normal physiological functions. 80% or more of intracellular protein degradation is dependent on the ubiquitin-proteasome system. PROTAC utilizes the cell's own protein destruction mechanism to remove specific targeted proteins from the cell. To date, PROTAC technology has become increasingly mature and can be used to target a variety of proteins, including scaffold proteins, transcription factors, enzymes, and regulatory proteins. Additionally, domide drugs are referred to as immunomodulatory drugs (IMiDs), which activate the E3 ubiquitin ligase complex formed with Cereblon (CRBN) to ubiquitinate transcription factors IKZF1 and IKZF3, subsequently recognized and degraded by the proteasome, thereby exerting cytotoxic effects on tumors. CRBN, as an important target for antitumor and immunomodulatory drugs, has been demonstrated to exhibit definitive therapeutic efficacy in various hematologic malignancies, skin diseases such as erythema nodosum leprosum, and autoimmune diseases such as systemic lupus erythematosus.

Therefore, the development of PROTAC and IMiD bifunctional molecules targeting IRAK4, by degrading IRAK4 and removing IRAK4, more thoroughly blocks all functions of IRAK4, thereby fundamentally achieving comprehensive inhibition of the IRAK4 signaling pathway, while possessing good CRBN modulatory effects to exert synergistic therapeutic actions, thus better exerting antitumor effects, enhancing clinical therapeutic effects.

SUMMARY

The present disclosure provides a maleate salt, a citrate salt, a p-toluenesulfonate salt, an oxalate salt, and a hydrobromide salt of a compound of formula (I); preferably, the maleate salt, the citrate salt, the p-toluenesulfonate salt, the oxalate salt, and the hydrobromide salt are in crystal form,

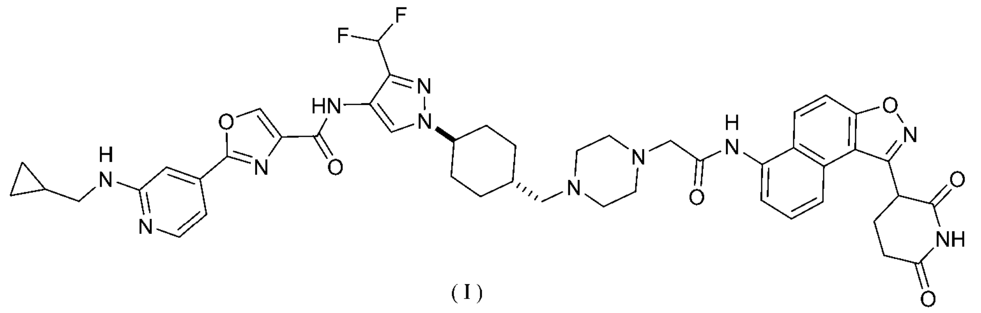

(I) .

In some embodiments of the present disclosure, the maleate salt of the compound of formula (I) is a compound of formula (II),

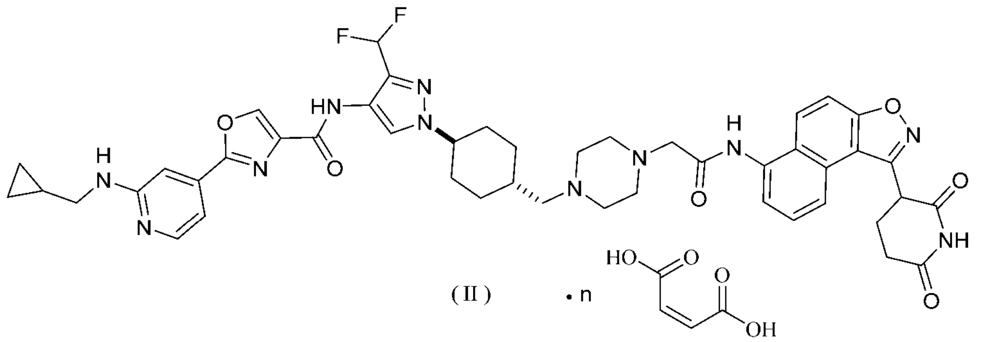

(II) • n ,

wherein n is 1.9 to 2.1; preferably 1.9, 2.0, or 2.1; more preferably 2.0.

In some embodiments of the present disclosure, the maleate salt of the compound of formula (I) has a molar ratio of the compound of formula (I) to maleic acid ranging from 1:1.9 to 1:2.1; preferably 1:1.9, 1:2.0, or 1:2.1; more preferably 1:2.0.

In some embodiments of the present disclosure, the compound of formula (II) is in crystal form.

In some embodiments of the present disclosure, the crystal form of the compound of formula (II) may exist in the crystal form of a solvate.

The present disclosure provides a crystal form A of the compound of formula (II), wherein the crystal form A of the compound of formula (II) has an X-ray powder diffraction pattern comprising characteristic diffraction peaks at the following 2θ angles: 6.67±0.20°, 9.52±0.20°, 17.03±0.20°, and 19.31±0.20°.

In some embodiments of the present disclosure, the X-ray powder diffraction pattern of the crystal form A of the compound of formula (II) comprises characteristic diffraction peaks at the following 20 angles: 6.67°, 9.52°, 17.03°, and 19.3 1°.

In some embodiments of the present disclosure, the X-ray powder diffraction (XRPD) pattern of the crystal form A of the compound of formula (II) is as shown in FIG. 1.

In some embodiments of the present disclosure, the X-ray powder diffraction (XRPD) pattern analysis data of the crystal form A of the compound of formula (II) is as shown in Table 1:

**Table 1.** XRPD pattern analysis data of the crystal form A of the compound of formula (II)

| No. | 2θ Angle (°) | *d*-Spacing (Å) | Relative Intensity (%) | No. | 2θ Angle (°) | *d*-Spacing (Å) | Relative Intensity (%) |
|---|---|---|---|---|---|---|---|
| 1 | 6.67 | 13.24 | 52.93 | 3 | 17.03 | 5.21 | 100.00 |
| 2 | 9.52 | 9.29 | 60.62 | 4 | 19.31 | 4.60 | 81.92 |

In some embodiments of the present disclosure, the crystal form A of the compound of formula (II) has a differential scanning calorimetry (DSC) curve showing endothermic peaks with peak temperatures at 153.3°C and 172.3°C.

In some embodiments of the present disclosure, the DSC pattern of the crystal form A of the compound of formula (II) is as shown in FIG. 2.

In some embodiments of the present disclosure, the crystal form A of the compound of formula (II) has a

thermogravimetric analysis (TGA) curve showing a weight loss of 6.42% at 150.0°C.

In some embodiments of the present disclosure, the TGA pattern of the crystal form A of the compound of formula (II) is as shown in FIG. 3.

In some embodiments of the present disclosure, the p-toluenesulfonate salt of the compound of formula (I) is a compound of formula (III),

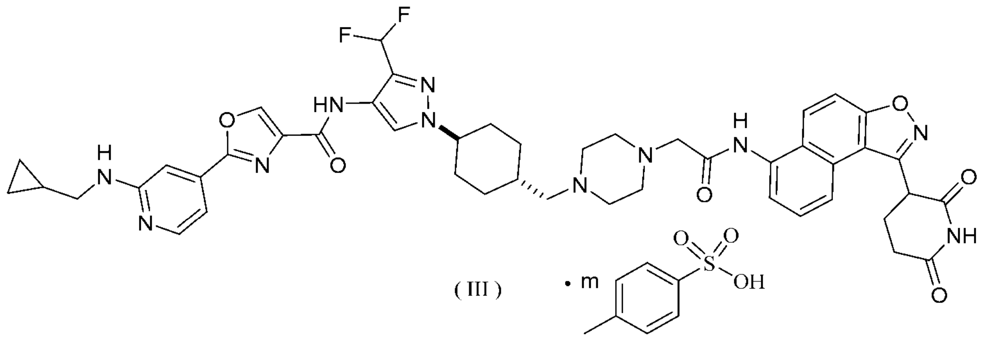

(III) • m

,

wherein m is 1.1 to 1.5; preferably 1.1, 1.2, 1.3, 1.4, or 1.5; more preferably 1.3.

In some embodiments of the present disclosure, the p-toluenesulfonate salt of the compound of formula (I) has a molar ratio of the compound of formula (I) to p-toluenesulfonic acid ranging from 1:1.1 to 1:1.5; preferably 1:1.1, 1:1.2, 1:1.3, 1:1.4, or 1:1.5; more preferably 1:1.3.

In some embodiments of the present disclosure, the compound of formula (III) is in crystal form.

In some embodiments of the present disclosure, the crystal form of the compound of formula (III) may exist in the crystal form of a solvate.

The present disclosure provides a crystal form A of the compound of formula (III), wherein the crystal form A of the compound of formula (III) has an X-ray powder diffraction pattern comprising characteristic diffraction peaks at the following 2θ angles: 10.61±0.20°, 12.06±0.20°, 15.79±0.20°, and 17.96±0.20°.

In some embodiments of the present disclosure, the X-ray powder diffraction pattern of the crystal form A of the compound of formula (III) comprises characteristic diffraction peaks at the following 2θ angles: 10.61°, 12.06°, 15.79°, and 17.96°.

In some embodiments of the present disclosure, the X-ray powder diffraction (XRPD) pattern of the crystal form A of the compound of formula (III) is as shown in FIG. 4.

In some embodiments of the present disclosure, the X-ray powder diffraction (XRPD) pattern analysis data of the crystal form A of the compound of formula (III) is as shown in Table 2:

**Table 2.** XRPD pattern analysis data of the crystal form A of the compound of formula (III)

| No. | 2θ Angle (°) | *d*-Spacing (Å) | Relative Intensity (%) | No. | 2θ Angle (°) | *d*-Spacing (Å) | Relative Intensity (%) |
|---|---|---|---|---|---|---|---|
| 1 | 10.61 | 8.34 | 80.14 | 3 | 15.79 | 5.61 | 37.68 |
| 2 | 12.06 | 7.34 | 100.00 | 4 | 17.96 | 4.94 | 65.03 |

In some embodiments of the present disclosure, the crystal form A of the compound of formula (III) has a differential scanning calorimetry curve showing endothermic peaks with peak temperatures at 78.5°C, 158.7°C, and 169.0°C.

In some embodiments of the present disclosure, the DSC pattern of the crystal form A of the compound of formula (III) is as shown in FIG. 5.

In some embodiments of the present disclosure, the crystal form A of the compound of formula (III) has a thermogravimetric analysis curve showing a weight loss of 5.16% at 150.0°C.

In some embodiments of the present disclosure, the TGA pattern of the crystal form A of the compound of formula (III) is as shown in FIG. 6.

In some embodiments of the present disclosure, the oxalate salt of the compound of formula (I) is a compound of formula (IV),

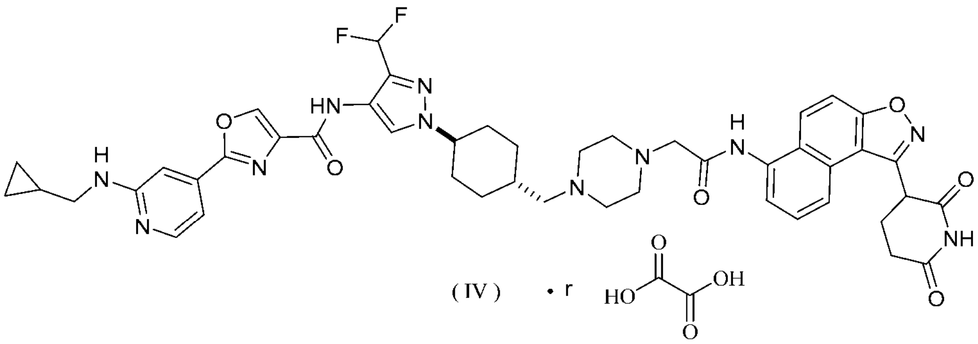

(IV) • r ,

wherein r is 2.1 to 2.6; preferably 2.1, 2.2, 2.3, 2.4, 2.5, or 2.6; more preferably 2.4.

In some embodiments of the present disclosure, the oxalate salt of the compound of formula (I) has a molar ratio of the compound of formula (I) to oxalic acid ranging from 1:2.1 to 1:2.6; preferably 1:2.1, 1:2.2, 1:2.3, 1:2.4, 1:2.5, or 1:2.6; more preferably 1:2.4.

In some embodiments of the present disclosure, the compound of formula (IV) is in crystal form.

In some embodiments of the present disclosure, the crystal form of the compound of formula (IV) may exist in the crystal form of a solvate.

The present disclosure provides a crystal form A of the compound of formula (IV), wherein the crystal form A of the compound of formula (IV) has an X-ray powder diffraction pattern comprising characteristic diffraction peaks at the following 2θ angles: 5.65±0.20°, 11.16±0.20°, and 19.49±0.20°.

In some embodiments of the present disclosure, the X-ray powder diffraction pattern of the crystal form A of the compound of formula (IV) comprises characteristic diffraction peaks at the following 2θ angles: 5.65±0.20°, 8.39±0.20°, 11.16±0.20°, 17.03±0.20°, 19.49±0.20°, 22.26±0.20°, and 23.09±0.20°.

In some embodiments of the present disclosure, the X-ray powder diffraction pattern of the crystal form A of the compound of formula (IV) comprises characteristic diffraction peaks at the following 2θ angles: 5.65°, 8.39°, 11.16°, 17.03°, 19.49°, 22.26°, and 23.09°.

In some embodiments of the present disclosure, the X-ray powder diffraction (XRPD) pattern of the crystal form A of the compound of formula (IV) is as shown in FIG. 7.

In some embodiments of the present disclosure, the X-ray powder diffraction (XRPD) pattern analysis data of the crystal form A of the compound of formula (IV) is as shown in Table 3:

**Table 3.** XRPD pattern analysis data of the crystal form A of the compound of formula (IV)

| No. | 2θ Angle (°) | *d*-Spacing (Å) | Relative Intensity (%) | No. | 2θ Angle (°) | *d*-Spacing (Å) | Relative Intensity (%) |
|---|---|---|---|---|---|---|---|
| 1 | 5.65 | 15.64 | 93.40 | 5 | 19.49 | 4.55 | 100.00 |
| 2 | 8.39 | 10.54 | 38.92 | 6 | 22.26 | 3.99 | 31.33 |
| 3 | 11.16 | 7.93 | 64.96 | 7 | 23.09 | 3.85 | 35.94 |
| 4 | 17.03 | 5.21 | 21.95 | | | | |

In some embodiments of the present disclosure, the crystal form A of the compound of formula (IV) has a differential scanning calorimetry curve showing an endothermic peak with a peak temperature at 216.9°C.

In some embodiments of the present disclosure, the DSC pattern of the crystal form A of the compound of formula (IV) is as shown in FIG. 8.

In some embodiments of the present disclosure, the crystal form A of the compound of formula (IV) has a thermogravimetric analysis curve showing a weight loss of 6.40% at 150.0°C.

In some embodiments of the present disclosure, the TGA pattern of the crystal form A of the compound of formula (IV) is as shown in FIG. 9.

In some embodiments of the present disclosure, the hydrobromide salt of the compound of formula (I) is a compound of formula (V),

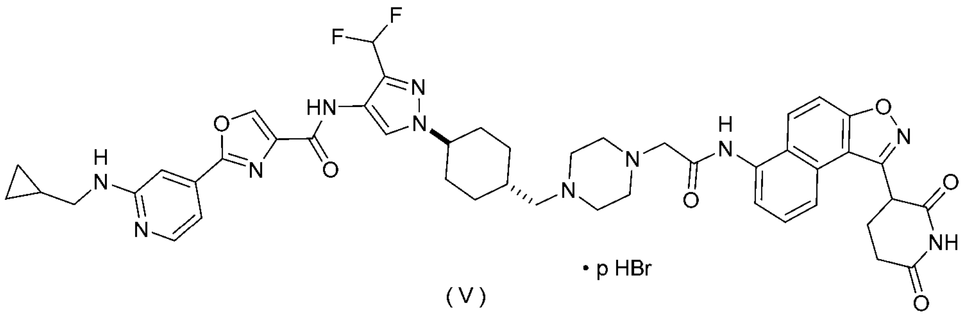

(V)

,

wherein p is 1.0 to 1.5; preferably 1.0, 1.1, 1.2, 1.3, 1.4, or 1.5; more preferably 1.2.

In some embodiments of the present disclosure, the hydrobromide salt of the compound of formula (I) has a molar ratio of the compound of formula (I) to hydrobromic acid ranging from 1:1.1 to 1:1.5; preferably 1:1.0, 1:1.1, 1:1.2, 1:1.3, 1:1.4, or 1:1.5; more preferably 1:1.2.

In some embodiments of the present disclosure, the compound of formula (V) is in crystal form.

In some embodiments of the present disclosure, the crystal form of the compound of formula (V) may exist in the crystal form of a solvate.

The present disclosure provides a crystal form A of the compound of formula (V), wherein the crystal form A of the compound of formula (V) has an X-ray powder diffraction pattern comprising characteristic diffraction peaks at the following 2θ angles: 6.51±0.20°, 8.94±0.20°, 17.87±0.20°, 19.88±0.20°, and 21.51±0.20°.

In some embodiments of the present disclosure, the X-ray powder diffraction pattern of the crystal form A of the compound of formula (V) comprises characteristic diffraction peaks at the following 2θ angles: 6.51±0.20°, 8.94±0.20°, 9.76±0.20°, 13.09±0.20°, 17.87±0.20°, 19.88±0.20°, 21.51±0.20°, and 27.11±0.20°.

In some embodiments of the present disclosure, the X-ray powder diffraction pattern of the crystal form A of the compound of formula (V) comprises characteristic diffraction peaks at the following 20 angles: 6.51°, 8.94°, 9.76°, 13.09°, 17.87°, 19.88°, 21.51°, 27.11°, and 29.37°.

In some embodiments of the present disclosure, the X-ray powder diffraction (XRPD) pattern of the crystal form A of the compound of formula (V) is as shown in FIG. 10.

In some embodiments of the present disclosure, the X-ray powder diffraction (XRPD) pattern analysis data of the crystal form A of the compound of formula (V) is as shown in Table 4:

**Table 4.** XRPD pattern analysis data of the crystal form A of the compound of formula (V)

| No. | 2θ Angle (°) | *d*-Spacing (Å) | Relative Intensity (%) | No. | 2θ Angle (°) | *d*-Spacing (Å) | Relative Intensity (%) |
|---|---|---|---|---|---|---|---|
| 1 | 6.51 | 13.58 | 41.14 | 6 | 19.88 | 4.47 | 100.00 |
| 2 | 8.94 | 9.89 | 50.53 | 7 | 21.51 | 4.13 | 50.80 |
| 3 | 9.76 | 9.06 | 33.71 | 8 | 27.11 | 3.29 | 23.56 |
| 4 | 13.09 | 6.76 | 22.15 | 9 | 29.37 | 3.04 | 24.11 |
| 5 | 17.87 | 4.96 | 49.01 | | | | |

In some embodiments of the present disclosure, the crystal form A of the compound of formula (V) has a differential scanning calorimetry curve showing endothermic peaks with peak temperatures at 64.7°C and 217.5°C.

In some embodiments of the present disclosure, the DSC pattern of the crystal form A of the compound of formula (V) is as shown in FIG. 11.

In some embodiments of the present disclosure, the crystal form A of the compound of formula (V) has a thermogravimetric analysis curve showing a weight loss of 4.06% at 150.0°C.

In some embodiments of the present disclosure, the TGA pattern of the crystal form A of the compound of formula (V) is as shown in FIG. 12.

In some embodiments of the present disclosure, the citrate salt of the compound of formula (I) is a compound of formula (VI),

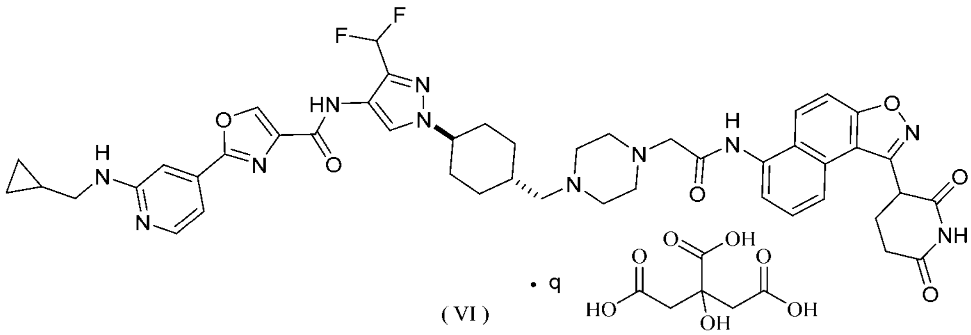

(VI) ,

wherein q is 2.1 to 2.3; preferably 2.1, 2.2, or 2.3; more preferably 2.2.

In some embodiments of the present disclosure, the citrate salt of the compound of formula (I) has a molar ratio of the compound of formula (I) to citric acid ranging from 1:2.1 to 1:2.3; preferably 1:2.1, 1:2.2, or 1:2.3; more preferably 1:2.2.

In some embodiments of the present disclosure, the compound of formula (VI) is in crystal form.

In some embodiments of the present disclosure, the crystal form of the compound of formula (VI) may exist in the crystal form of a solvate.

The present disclosure provides a crystal form A of the compound of formula (VI), wherein the crystal form A of the compound of formula (VI) has an X-ray powder diffraction pattern comprising characteristic diffraction peaks at the following 2θ angles: 7.63±0.20° and 18.46±0.20°.

In some embodiments of the present disclosure, the X-ray powder diffraction pattern of the crystal form A of the compound of formula (VI) comprises characteristic diffraction peaks at the following 2θ angles: 7.63° and 18.46°.

In some embodiments of the present disclosure, the X-ray powder diffraction (XRPD) pattern of the crystal form A of the compound of formula (VI) is as shown in FIG. 13.

In some embodiments of the present disclosure, the X-ray powder diffraction (XRPD) pattern analysis data of the crystal form A of the compound of formula (VI) is as shown in Table 5:

**Table 5.** XRPD pattern analysis data of the crystal form A of the compound of formula (VI)

| No. | 2θ Angle (°) | *d*-Spacing (Å) | Relative Intensity (%) | No. | 2θ Angle (°) | *d*-Spacing (Å) | Relative Intensity (%) |
|---|---|---|---|---|---|---|---|
| 1 | 7.63 | 11.59 | 49.08 | 2 | 18.46 | 4.81 | 100.00 |

In some embodiments of the present disclosure, the crystal form A of the compound of formula (VI) has a differential scanning calorimetry curve showing endothermic peaks with peak temperatures at 77.6°C, 159.4°C, and 184.2°C.

In some embodiments of the present disclosure, the DSC pattern of the crystal form A of the compound of formula (VI) is as shown in FIG. 14.

In some embodiments of the present disclosure, the crystal form A of the compound of formula (VI) has a thermogravimetric analysis curve showing a weight loss of 7.05% at 150.0°C.

In some embodiments of the present disclosure, the TGA pattern of the crystal form A of the compound of formula (VI) is as shown in FIG. 15.

The present disclosure also provides a use of the compound of formula (II), the compound of formula (III), the compound of formula (IV), the compound of formula (V), the compound of formula (VI), the crystal form A of the compound of formula (II), the crystal form A of the compound of formula (III), the crystal form A of the compound of formula (IV), the crystal form A of the compound of formula (V), or the crystal form A of the compound of formula (VI) in the manufacture of a medicament for treating diffuse large B-cell lymphoma.

## Technical Effect

The compound of the present disclosure exhibits excellent degradation effects on the target proteins IRAK4, IKZF1, and IKZF3, as well as excellent inhibition effects on cell proliferation in lymphoma cell lines OCI-LY10, TMD-8, and SU-DHL-2. The compound of the present disclosure exhibits significant tumor inhibitory effects with dose dependency, along with good pharmacokinetic properties and oral absorption rates, making it suitable for use as a medicament.

**Definition and Description**

Unless otherwise specified, the following terms and phrases, when used herein, have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

For any given crystal form, the relative intensities of diffraction peaks may vary due to factors such as preferred orientation caused by crystal morphology, which is well-known in the field of crystallography. Where preferred orientation effects exist, the peak intensities are altered, but the diffraction peak positions of the crystal form remain unchanged. Furthermore, for any given crystal form, slight deviations in peak positions may exist, which is also well-known in the field of crystallography. For example, due to temperature fluctuations during sample analysis, sample movement, or instrument calibration, peak positions may shift, with measurement errors for 2θ values sometimes around ±0.20°. Therefore, it is well-known to those skilled in the art that such errors should be taken into account when determining each crystal structure.

DSC measures the transition temperature at which a crystal absorbs or releases heat due to changes in its crystal structure or the melting of the crystal. For the same crystal form of the same compound, in consecutive analyses, the thermal transition temperature and melting point typically vary within approximately 5°C or 3°C. When we state that a compound exhibits a given DSC peak or melting point, it refers to the DSC peak or melting point ±5°C or ±3°C. DSC provides an auxiliary method for distinguishing different crystal forms. Different crystal forms can be identified based on their distinct transition temperature characteristics. It should be noted that for mixtures, their DSC peaks or melting points may vary over a broader range. Furthermore, since decomposition accompanies the melting process of the substance, the melting temperature is related to the heating rate.

Unless otherwise specified, in the DSC pattern, the upward peak is exothermic.

For the same crystal form, the TGA weight loss temperature may vary due to factors such as the measuring instrument, method/conditions, *etc.* For any specific crystal form, the weight loss temperature may have an error, which can be approximately ±5°C or approximately ±3°C.

It should be noted that during the manufacture of drug crystal forms, it is difficult to avoid the formation of co crystals between drug molecules and solvent molecules due to external conditions and internal factors, resulting in residual solvent molecules in the solid material, thereby forming solvates, including stoichiometric solvates and non-stoichiometric solvates. The solvates are all included within the scope of the present disclosure.

The term "pharmaceutically acceptable excipient" refers to inert substances administered together with the active ingredient to facilitate the administration of the active ingredient, including but not limited to any glidants, sweetening agents, diluents, preservatives, dyes/colorants, flavor enhancers, surfactants, wetting agents, dispersants, disintegrants, suspending agents, stabilizers, isotonic agents, solvents, or emulsifiers approved by the National Medical Products Administration for use in humans or animals (*e.g.,* livestock).

The term "crystalline composition" refers to a mixture consisting of the crystal form of the compound of formula (I) of the present disclosure with other crystal forms or amorphous forms of the compound or other impurities. For example, the crystalline composition of the compound of formula (I) comprises, in addition to the crystal form of the compound of formula (I), other crystal forms or amorphous forms of compound 1 or other impurities.

The term "pharmaceutical composition" refers to a mixture of one or more compounds of the present disclosure or salts thereof with pharmaceutically acceptable excipients. The purpose of the pharmaceutical composition is to facilitate the administration of the compounds of the present disclosure to an organism.

The therapeutic dosage of the compound of the present disclosure may be determined based on, for example, the specific use of the treatment, the mode of administration of the compound, the health and condition of the patient, and the judgment of the prescribing physician. The proportion or concentration of the compound of the present disclosure in the pharmaceutical composition may not be fixed and depends on a variety of factors including dosage, chemical properties (e.g., hydrophobicity), and route of administration.

The term "treatment" refers to the administration of the compound or formulation of the present disclosure to ameliorate or eliminate a disease or one or more symptoms associated with the disease, and includes:

(i) inhibiting a disease or disease state, *i.e.,* arresting its development;
(ii) alleviating a disease or disease state, *i.e.,* causing its regression.

The term "therapeutically effective amount" refers to an amount of the compound of the present disclosure for (i) treating a specific disease, condition, or disorder; (ii) alleviating, ameliorating, or eliminating one or more symptoms of a specific disease, condition, or disorder, or (iii) preventing or delaying the onset of one or more symptoms of a specific disease, condition, or disorder described herein. The amount of the compound of the present disclosure composing the "therapeutically effective amount" varies depending on the compound, the disease state and its severity, the mode of administration, and the age of the mammal to be treated, but can be routinely determined by those skilled in the art based

on their knowledge and the present disclosure.

Unless the context requires otherwise, throughout the specification and claims thereafter, the term "comprise" and English variations thereof, such as "comprises" and "comprising", are to be construed in an open and inclusive sense, *i.e.,* "including, but not limited to".

Reference throughout the specification to "some embodiment" or "an embodiment" or "another embodiment" or "certain embodiments" means that at least one embodiment includes specific reference elements, structures, or characteristics described in connection with the embodiment. Accordingly, the phrase "in some embodiment" or "in an embodiment" or "in another embodiment" or "in certain embodiments" appearing in various places throughout the specification does not necessarily all refer to the same embodiment. In addition, the specific elements, structures, or characteristics may be combined in any suitable manner in one or more than one embodiment.

It should be understood that the singular forms "a", "an", and "the" used in the specification and appended claims of the present disclosure include plural referents unless the context clearly dictates otherwise. Thus, for example, reference to a reaction including "a catalyst" includes one catalyst, or two or more catalysts. It should also be understood that the term "or" is generally used in its sense including "and/or" unless the context clearly dictates otherwise.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include, but are not limited to, the examples of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, it is sometimes necessary for those skilled in the art to modify or select the synthetic steps or reaction processes on the basis of the existing embodiments.

Unless otherwise specified, the absolute configuration of a stereogenic center is represented by a wedged solid bond (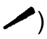) and a wedged dashed bond (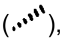), and the relative configuration is represented by a straight solid bond (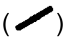) and a straight dashed bond (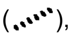), for example, *trans*-1,4-disubstituted cyclohexane is represented by

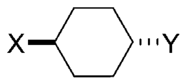

or

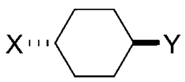

,

and *cis*-1,4-disubstituted cyclohexane is represented by

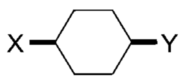

or

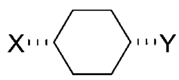

.

The present disclosure is described in detail by the examples below, but these examples do not imply any restrictions on the present disclosure.

All solvents used in the present disclosure are commercially available and can be used without further purification.

Compounds are named manually or using ChemDraw® software, while commercially available compounds adopt the supplier catalog names.

The following abbreviations are used in the present disclosure: RT represents room temperature; MeOH represents methanol; EtOH represents ethanol; IPA represents isopropanol; Acetone represents 2-propanone; MIBK represents methyl isobutyl ketone; EtOAc represents ethyl acetate; IPAc represents isopropyl acetate; MTBE represents methyl *tert*-butyl ether; THF represents tetrahydrofuran; 2-Me THF represents 2-methyltetrahydrofuran; DCM represents dichloromethane; $CHCl_3$ represents chloroform; Toluene represents methylbenzene; n-Heptane represents dipropylmethane; DMSO represents dimethyl sulfoxide; DMAc represents *N,N*-dimethylacetamide; NMP represents *N*-methylpyrrolidone; $H_2O$ represents water; 1,4-Dioxane represents 1,4-dioxacyclohexane; ACN represents acetonitrile.

BRIEF DESCRIPTION OF THE DRAWINGS

FIG. 1 shows the X-ray powder diffraction (XRPD) pattern of the crystal form A of the compound of formula (II);
FIG. 2 shows the DSC pattern of the crystal form A of the compound of formula (II);
FIG. 3 shows the TGA pattern of the crystal form A of the compound of formula (II);
FIG. 4 shows the X-ray powder diffraction (XRPD) pattern of the crystal form A of the compound of formula (III);
FIG. 5 shows the DSC pattern of the crystal form A of the compound of formula (III);
FIG. 6 shows the TGA pattern of the crystal form A of the compound of formula (III);
FIG. 7 shows the X-ray powder diffraction (XRPD) pattern of the crystal form A of the compound of formula (IV);
FIG. 8 shows the DSC pattern of the crystal form A of the compound of formula (IV);
FIG. 9 shows the TGA pattern of the crystal form A of the compound of formula (IV);
FIG. 10 shows the X-ray powder diffraction (XRPD) pattern of the crystal form A of the compound of formula (V);
FIG. 11 shows the DSC pattern of the crystal form A of the compound of formula (V);
FIG. 12 shows the TGA pattern of the crystal form A of the compound of formula (V);
FIG. 13 shows the X-ray powder diffraction (XRPD) pattern of the crystal form A of the compound of formula (VI);
FIG. 14 shows the DSC pattern of the crystal form A of the compound of formula (VI);
FIG. 15 shows the TGA pattern of the crystal form A of the compound of formula (VI);
FIG. 16 shows the solution NMR spectrum of the crystal form of the compound of formula (II);
FIG. 17 shows the solution NMR spectrum of the crystal form A of the compound of formula (III);
FIG. 18 shows the solution NMR spectrum of the crystal form A of the compound of formula (IV);
FIG. 19 shows the solution NMR spectrum of the crystal form A of the compound of formula (V);
FIG. 20 shows the solution NMR spectrum of the crystal form of the compound of formula (VI).

**Instruments and Analytical Methods**

**1.1 X-ray powder diffraction (X-ray powder diffractometer, XRPD) method of the present disclosure**

Instrument model: PANalytacal X-ray Powder Diffractometer
Test method: Approximately 10 to 20 mg of the sample is used for XRPD detection.
The detailed XRPD parameters are as follows:
X-ray tube: Cu, kα, (λ = 1.540598 Å, λ = 1.544426 Å).
X-ray tube voltage: 45 kV, X-ray tube current: 40 mA
Divergence slit: 1/8°
Scan mode: continuous
Scanning range (°2Theta): 3-40
Scanning time per step (s): 46.7
Scanning step size (°2Theta): 0.0263
Test duration: approximately 5 min

**1.2 Differential scanning calorimetry (differential scanning calorimeter, DSC) method of the present disclosure**

Instrument model: TA 2500 Differential Scanning Calorimeter
Test method: A sample (approximately 1 mg) is placed in a DSC aluminum crucible for testing. Under a 50 mL/min $N_2$ atmosphere, the sample is heated from 25°C (room temperature) to 300°C (or 350°C) at a heating rate of 10°C/min.

**1.3 Thermogravimetric analysis (thermal gravimetric analyzer, TGA) method of the present disclosure**

Instrument model: TA 5500 Thermal Gravimetric Analyzer
Test method: The sample (2 to 5 mg) is placed in a TGA platinum crucible for testing. Under a 25 mL/min $N_2$

atmosphere, the sample is heated from room temperature to 350°C or until a 20% weight loss is achieved, at a heating rate of 10°C/min.

**1.4 Dynamic vapor sorption (DVS) method of the present disclosure**

Instrument model: DVS Intrinsic Plus Dynamic Vapor Sorption Analyzer from SMS (Surface Measurement Systems)
Test conditions: The sample (10 to 20 mg) is placed in a DVS sample pan for testing.
The detailed DVS parameters are as follows:
Protective gas and flow rate: $N_2$, 200 mL/min
Temperature: 25°C
Equilibrium: dm/dt = 0.002%/min (minimum: 10 min, maximum: 180 min)
Drying: drying for 120 min under 0% RH
RH (%) range: 0% - 95%
RH (%) gradient: 10% (0%RH-90%RH, 90%RH-0%RH); 5% (90%RH-95%RH, 95%RH-90%RH)
The hygroscopicity evaluation is classified as follows in Table 6:

Table 6: Evaluation of hygroscopicity

| **Classification of Hygroscopicity** | ΔW% |
|---|---|
| Deliquescence | Absorption of Sufficient Water to Form a Liquid |
| Highly Hygroscopic | ΔW% ≥ 15% |
| Hygroscopic | 15% > ΔW% ≥ 2% |
| Slightly Hygroscopic | 2% > ΔW% ≥ 0.2% |
| Non-Hygroscopic or Virtually Non-Hygroscopic | ΔW% < 0.2% |
| Note: ΔW% represents the hygroscopic weight gain of the test sample at 25±1°C and 80±2% RH. | |

**1.5 Ultra-high performance liquid chromatography/ion chromatography (UPLC/IC) method of the present disclosure**

Instrument model: Waters H-Class Ultra-High Performance Liquid Chromatograph and Ion Chromatography
The detailed UPLC parameters are as follows:
Chromatographic column: Xbridge C18, 4.6×150 mm, 5 μm
Mobile phase: A: 0.1% TFA in $H_2O$; B: 0.1% TFA in Acetonitrile
Gradient: 0.0 min, 10%B; 6.0 min, 90%B; 10.0 min, 90%B; 10.1 min, 10%B; 15.0 min, 10%B
Run time: 15.0 min
Mobile phase flow rate: 0.0 min
Injection volume: 1.0 mL/min
Detection wavelength: 3 μL
Column temperature: UV at 226 nm
Injector temperature: 25°C
Diluent: ACN/$H_2O$ =1:1 (v/v)
The detailed IC parameters are as follows:
Chromatographic column: Dionex IonPac™ AS18 RFIC™ 4×250 mm Analytical
Mobile phase: 25 mM NaOH
Injection volume: 25 μL
Flow rate: 1.0 mL/min
Temperature: 35°C
Column temperature: 35°C
Current: 80 mA
Run time: 16 mins

**1.6 Solution NMR method of the present disclosure**

Instrument model: Bruker 400 M NMR Spectrometer

Solvent: DMSO-$d_6$ or deuterated methanol

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

**Example 1: Preparation of the compound of formula (I)**

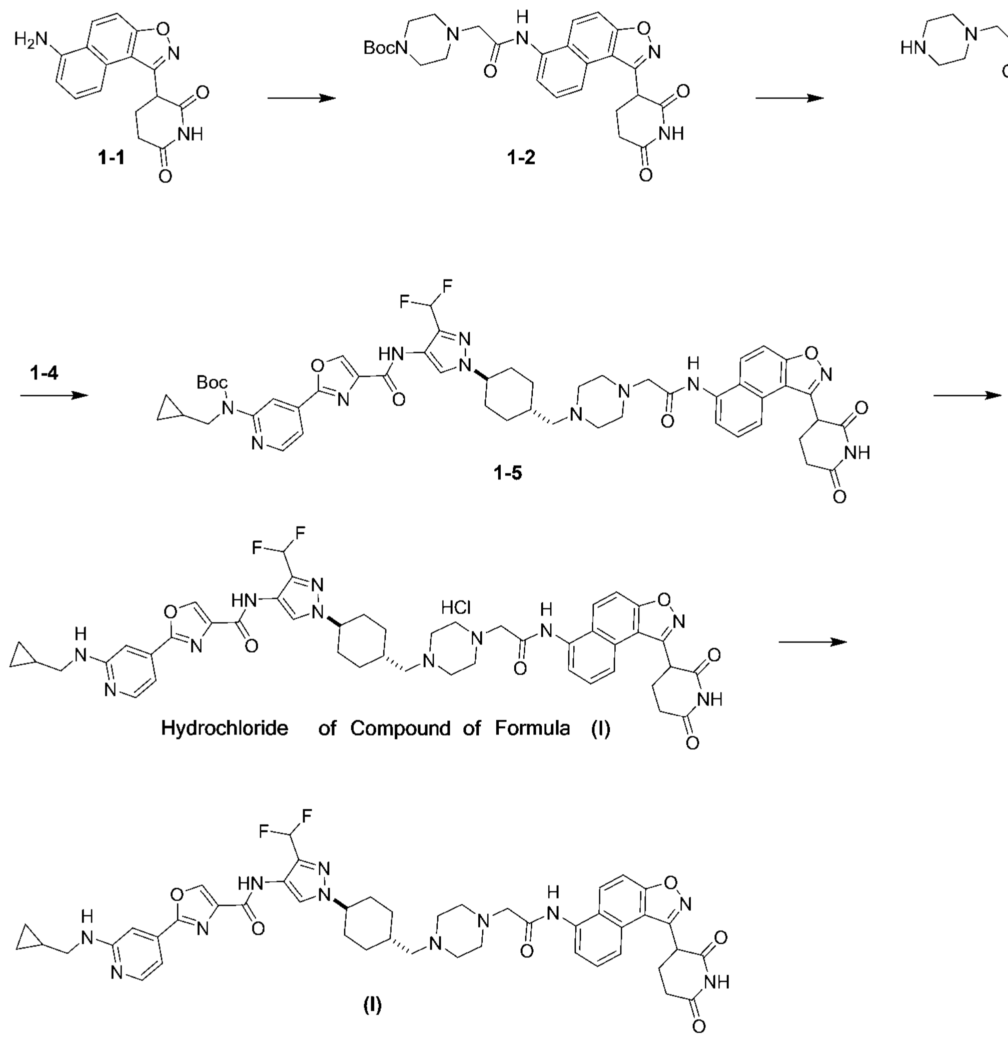



1-1 1-2 1-3 1-5

Hydrochloride of Compound of Formula (I)

(I)

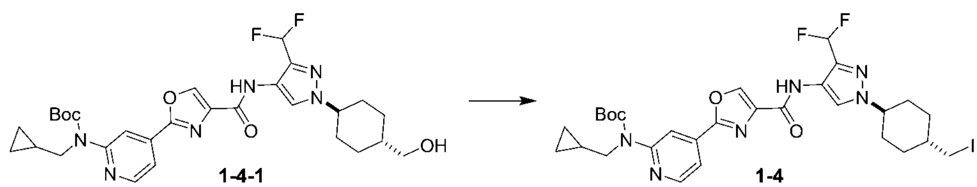

1-4-1 1-4

Step 1: Synthesis of compound **1-2**

Under room temperature and a nitrogen atmosphere, 2-(4-(*tert*-butoxycarbonyl)piperazin-1-yl)acetic acid (115.98 g, 474.75 mmol) was dissolved in *N,N*-dimethylformamide (1 L), followed by the addition of *N,N*-diisopropylethylamine (163.62 g, 1.27 mol) and 2-(7-azabenzotriazol-1-yl)-*N*,*N*,*N'*,*N'*-tetramethyluronium hexafluorophosphate (180.51 g, 474.75 mmol). The reaction mixture was stirred at room temperature for 0.5 hours, and then the hydrochloride of compound **1-1** (105 g, 316.5 mmol) was added. The reaction mixture was stirred at room temperature for 12 hours. After the reaction was completed, the reaction mixture was poured into ice water (5 L) and extracted with ethyl acetate (4×1 L). The organic phases were combined, washed with saturated brine (3×1 L), dried over anhydrous sodium sulfate, filtered, and the filtrate was concentrated under reduced pressure to remove the solvent. The residue was dissolved in a mixed solvent of ethyl acetate and dichloromethane (volume ratio 1:1, 1.5 L), stirred at room temperature for 1 hour, during which solids gradually precipitated. The majority of dichloromethane was removed by concentration under reduced pressure, methyl *tert*-butyl ether (1.5 L) was added, and the mixture was stirred at room temperature for 12 hours, filtered, and the filter cake was collected. Ethyl acetate (1.5 L) was added to the filter cake, stirred at room temperature for 2 hours, filtered, and the filter cake was rinsed with ethyl acetate (200 mL×2). The filter cake was collected and dried under vacuum to obtain compound **1-2.** MS-ESI *m/z:* 522.3 $[M+H]^+$. $^1H$ NMR (400 MHz, DMSO_$d_6$) δ: 11.14 (s, 1H), 10.13 (s, 1H), 8.19 (d, *J*=9.6 Hz, 1H), 8.11 (d, *J*=8.0 Hz, 1H), 8.00 (d, *J*=9.26 Hz, 1H), 7.80-7.68 (m, 2H), 5.09 (dd, *J*=4.8 Hz, 11.2 Hz, 1H), 3.52-3.39 (m, 4H), 3.32 (s, 2H), 2.93-2.78 (m, 1H), 2.70-2.55 (m, 6H), 2.45-2.35 (m, 1H), 1.42 (s, 9H).

Step 2: Synthesis of the trifluoroacetate salt of compound **1-3**

At room temperature, compound **1-2** (153 g, 293.35 mmol) was dissolved in dichloromethane (1.2 L), and trifluoroacetic acid (462 g, 4.05 mol) was slowly added dropwise. The reaction mixture was stirred at room temperature for 12 hours. After the reaction was completed, the reaction mixture was concentrated under reduced pressure to remove the solvent to obtain the trifluoroacetate salt of compound **1-3.** MS-ESI *m/z:* 422.1 $[M+H]^+$.

Step 3: Synthesis of compound **1-4**

Under room temperature and a nitrogen atmosphere, compound **1-4-1** (153 g, 260.81 mmol) was dissolved in dichloromethane (3 L), followed by the addition of triphenylphosphine (82.09 g, 312.97 mmol) and imidazole (26.63 g, 319.22 mmol). The reaction mixture was cooled to 0°C in an ice bath, and elemental iodine (86.06 g, 339.06 mmol) was added in batches. The reaction mixture was warmed to room temperature and stirred for 16 hours. After the reaction was completed, a saturated sodium sulfite solution (1 L) was slowly added to the reaction mixture, stirred for 20 minutes, and then the phases were separated. The aqueous phase was extracted with dichloromethane (3×3 L). The organic phases were combined, washed with saturated brine (2×2 L), dried over anhydrous sodium sulfate, filtered, and the filtrate was concentrated under reduced pressure to remove the solvent. Methyl *tert*-butyl ether (600 mL) was added to the residue, and the mixture was stirred at room temperature for 2 hours. The mixture was filtered, and the filter cake was rinsed with methyl *tert*-butyl ether (50 mL×2). The filtrate was collected. Anhydrous magnesium chloride (80 g) was added to the filtrate, and the mixture was stirred at room temperature for 8 hours. The mixture was filtered, and the filter cake was rinsed with methyl *tert*-butyl ether (50 mL×2). The filtrate was collected and concentrated under reduced pressure to obtain compound **1-4.** MS-ESI *m/z:* 697.0 $[M+H]^+$.

Step 4: Synthesis of compound **1-5**

Under room temperature and a nitrogen atmosphere, the trifluoroacetate salt of compound **1-3** (129.35 g, 157.64 mmol) was dissolved in acetonitrile (1.3 L), followed by the addition of *N,N*-diisopropylethylamine (158.46 g, 1.23 mol) and compound **1-4** (122 g, 175.16 mmol). The reaction mixture was heated to 80°C and stirred for 12 hours. After the reaction was completed, part of the solvent was removed by concentration under reduced pressure, and ethyl acetate (500 mL) and water (500 mL) were added. The phases were separated, and the aqueous phase was extracted with ethyl acetate (500 mL×3). The organic phases were combined, washed with saturated brine (300 mL), dried over anhydrous sodium sulfate, filtered, and the filtrate was concentrated under reduced pressure to remove the solvent. The residue was dissolved in a mixed solvent of ethyl acetate and acetonitrile (volume ratio 9:1, 700 mL), stirred at room temperature for 2 hours, filtered, and the filter cake was rinsed with a mixed solvent of ethyl acetate and acetonitrile (volume ratio 9:1, 30 mL×3).The filter cake was collected and dried under vacuum to obtain compound **1-5.** MS-ESI *m/z:* 990.4 $[M+H]^+$.

Step 5: Synthesis of the hydrochloride of the compound of formula (I)

Compound **1-5** (80 g, 80.8 mmol) was dissolved in ethyl acetate (100 mL) under room temperature and a nitrogen

atmosphere, then a solution of hydrogen chloride in ethyl acetate (4 M, 500 mL) was slowly added, and the reaction mixture was stirred at room temperature for 16 hours. After the reaction was completed, the mixture was filtered, and the filter cake was rinsed with ethyl acetate (50 mL×3).The filter cake was collected. Acetonitrile (350 mL) was added to the filter cake, and the mixture was stirred at room temperature for 2 hours. The mixture was filtered, and the filter cake was washed with acetonitrile (30 mL×3).The filter cake was collected and dried under vacuum to obtain the hydrochloride of the compound of formula (I). MS-ESI *m/z:* 890.2 $[M+H]^+$. $^1$H NMR (400 MHz, DMSO_$d_6$) δ: 11.59 (s, 1H), 11.14 (s, 1H), 11.05 (s, 1H), 9.97 (s, 1H), 9.59 (s, 1H), 9.15 (s, 1H), 8.45 (d, *J*=9.2 Hz, 1H), 8.23-8.12 (m, 2H), 8.06 (d, J=6.0 Hz, 1H), 7.99 (d, *J*=9.2 Hz, 1H), 7.81-7.73 (m, 2H), 7.70 (s, 1H), 7.29 (d, *J*=6.8 Hz, 1H), 7.22 (t, *J*=54.4 Hz, 1H), 5.11 (dd, *J*=4.0 Hz, 11.2 Hz, 1H), 4.42 (s, 2H), 4.31-4.20 (m, 1H), 3.81 (s, 6H), 3.47 (s, 2H), 3.39 (d, *J*=6.4 Hz, 2H), 3.11 (s, 2H), 2.94-2.78 (m, 1H), 2.72-2.54 (m, 2H), 2.45-2.35 (m, 1H), 2.17-2.02 (m, 5H), 2.01-1.90 (m, 1H), 1.88-1.74 (m, 2H), 1.29-1.09 (m, 2H), 0.61-0.50 (m, 2H), 0.39-0.30 (m, 2H).

Step 6: Synthesis of the compound of formula (I)

Under room temperature and a nitrogen atmosphere, the hydrochloride of the compound of formula (I) (10 g) was dissolved in a mixed solvent of isopropanol (75 mL) and water (300 mL). The mixed solution was added dropwise to a solution of imidazole (3.43 g, 50.4 mmol) in water (300 mL), stirred at room temperature for 15 hours, and filtered. The filter cake was washed with water (50 mL×2) and isopropanol (50 mL), collected, and dried under vacuum to obtain the compound of formula (I) (free base).

**Example 2: Preparation of the crystal form A of the compound of formula (II)**

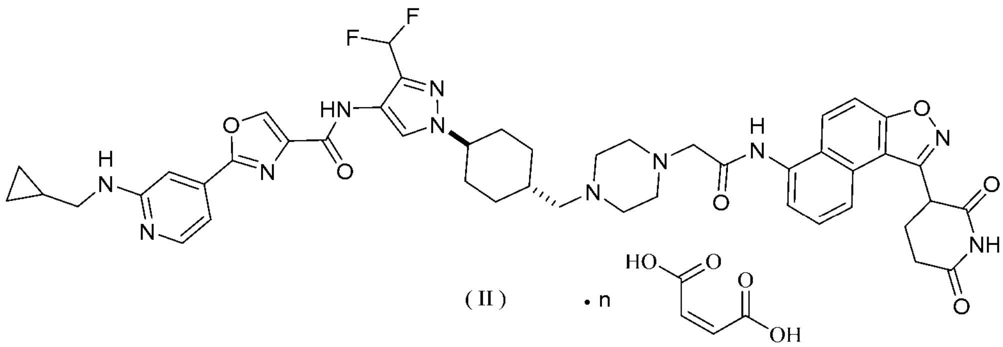

(II) • n

20 mg of the compound of formula (I) was taken, and 0.5 mL of tetrahydrofuran was added, and then 2.0 equivalents of maleic acid was added under stirring. The reaction mixture was stirred at 25°C for 72 hours to obtain a solid. The sample was separated by centrifugation to obtain the crystal form A of the compound of formula (II). $^1$H NMR results are shown in FIG. 16, with a molar ratio of acid to base in the sample being 2.0, *i.e.,* n = 2.0.

**Example 3: Preparation of the crystal form A of the compound of formula (III)**

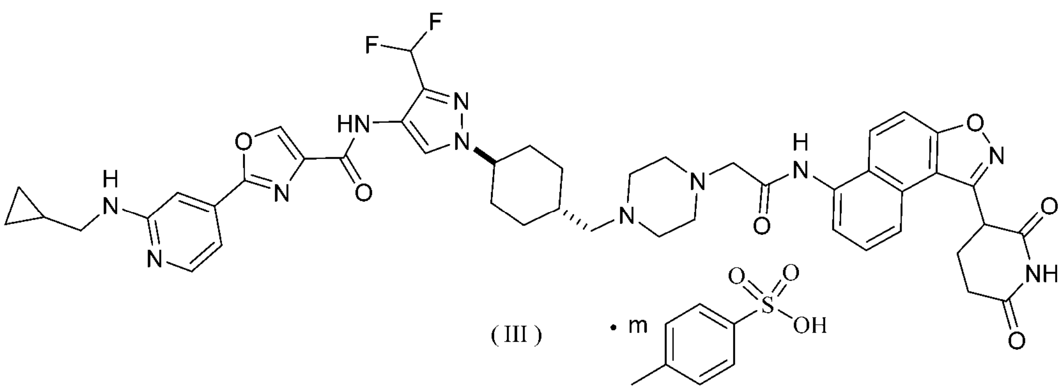

(III) • m

20 mg of the compound of formula (I) was taken, and 0.5 mL of tetrahydrofuran was added, and then 1.0 equivalents of p-toluenesulfonic acid was added under stirring. The reaction mixture was stirred at 25°C for 48 hours to obtain a solid. The sample was separated by centrifugation to obtain the crystal form A of the compound of formula (III). $^1$H NMR results are shown in FIG. 17, with a molar ratio of acid to base in the sample being 1.3, *i.e.,* m = 1.3.

**Example 4: Preparation of the crystal form A of the compound of formula (IV)**

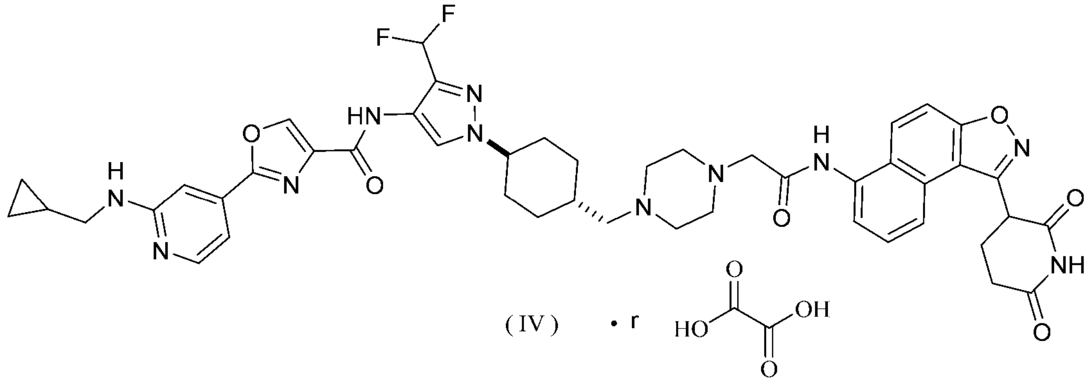

(IV) • r

20 mg of the compound of formula (I) was taken, and 0.5 mL of tetrahydrofuran was added, and then 2.0 equivalents of oxalic acid was added under stirring. The reaction mixture was stirred at 25°C for 48 hours to obtain a solid. The sample was separated by centrifugation to obtain the crystal form of the compound of formula (IV). $^1$H NMR results are as shown in FIG. 18, and UPLC/IC results show a molar ratio of acid to base of 2.4, *i.e.,* r = 2.4.

**Example 5: Preparation of the crystal form A of the compound of formula (V)**

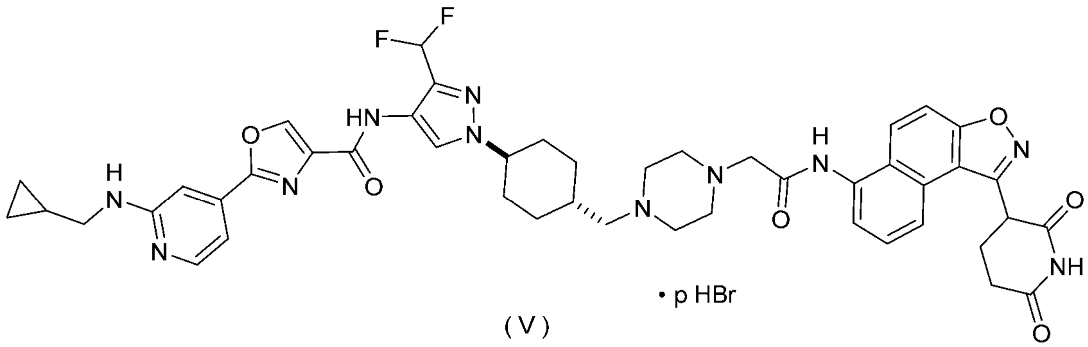

• p HBr

(V)

20 mg of the compound of formula (I) was taken, and 0.5 mL of tetrahydrofuran was added, followed by the addition of 1.0 equivalent of hydrobromic acid under stirring. The reaction mixture was stirred at 25°C for 48 hours to obtain a solid. The sample was separated by centrifugation to obtain the crystal form A of the compound of formula (V). $^1$H NMR results are shown in FIG. 19, and UPLC/IC results show a molar ratio of acid to base of 1.2, *i.e.,* p = 1.2.

**Example 6: Preparation of the crystal form A of the compound of formula (VI)**

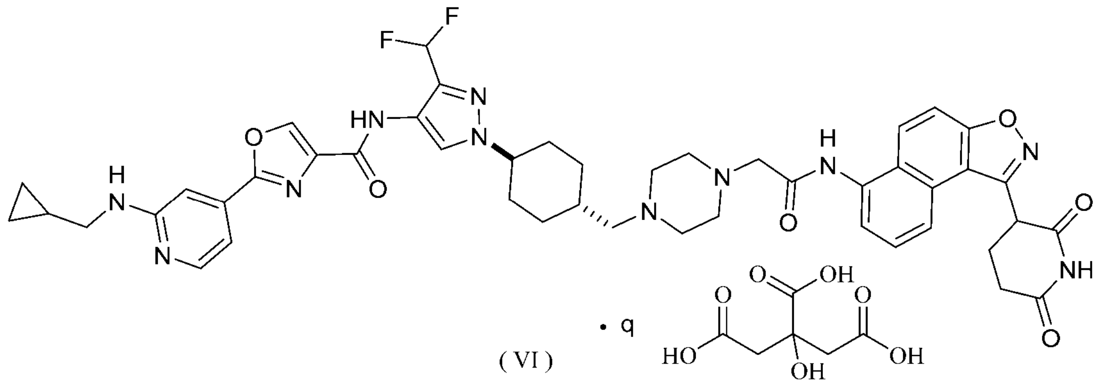

(VI)

20 mg of the compound of formula (I) was taken, and 0.5 mL of tetrahydrofuran was added, followed by the addition of 2.0 equivalents of citric acid under stirring. The reaction mixture was stirred at 25°C for 48 hours to obtain a solid. The sample was separated by centrifugation to obtain the crystal form A of the compound of formula (VI). $^{1}$H NMR results are shown in FIG. 20, with a molar ratio of acid to base in the sample being 2.2, *i.e.,* q = 2.2.

**Bioassay**

**Experimental Example 1: Evaluation of the target protein degradation effect of the compound of formula (I) in K562 IRAK4-HiBiT cells**

**Objective:** In this experiment, the degradation effect of the compound of formula (I) on the target protein IRAK4 in K562 IRAK4-HiBiT cells was detected.

**Experimental materials:**

1. Cells and culture medium

Cells: K562 IRAK4-HiBiT cells
Culture medium: RPMI 1640 + 10% fetal bovine serum + 2 mM glutamine + 1 mM sodium pyruvate + penicillin/-streptomycin
Positive control: 1000 nM; negative control: 0.1% DMSO

Table **7.** Reagents and consumables

| Reagents and Consumables | Manufacturer | Cat. No. |
|---|---|---|
| RPMI 1640 | Gibco | Cat# 31800 |
| Fetal Bovine Serum (FBS) | Biosera | #FB-1058/500 |
| Penicillin/Streptomycin | Biosera | #LM-A4118 |
| DPBS | Invitrogen | #14190 |
| 0.25% Trypsin/EDTA Solution | Invitrogen | #25200 |
| Glutamine | Invitrogen | Cat# 35050061 |
| Sodium Pyruvate | Invitrogen | Cat# 11360070 |
| Nano-Glo® HiBiT Assay Kit | Promega | Promega# N3040 |
| 384-Well White Plate, Flat Bottom | Corning | Cat# 3570 |
| 384_LDV Compound Plate | Labcyte | Cat# LP-0200 |

Table **8.** Instruments

| Instrument Name | Manufacturer | Instrument Model |
|---|---|---|
| ECHO Pipette | Labcyte | Echo 550 |
| Bravo Automated Liquid Handling Platform | Agilent | 16050-101 |
| Envision Plate Reader | PerkinElmer | 2104 |
| Autosampler | Thermo Fisher | Multidrop Combi |
| Cell Counter | Thermo | Countess II FL |

**Experimental scheme:**

**Day 1**

1. Compound preparation

(1) The test compound in powder form was dissolved in DMSO to 10 mM as a storage concentration, and 9 μL of the 10 mM test compound was manually pipetted to column 1 and column 13 of an LDV plate using a pipette.
(2) 6 μL of DMSO was added to columns 2 to 12 and 14 to 24 using Multidrop Combi.
(3) The test compound was 3-fold diluted (3 μL + 6 μL) from columns 1 to 11 and 13 to 23 using Bravo.
(4) 25 nL of compound solution (columns 1 to 24 of the LDV plate) was transferred to an assay plate using Echo according to the plate layout.
(5) 25 nL of 1 mM positive control solution was transferred to the assay plate as a 100% degradation control (*i.e.,* LC, HPE), and 25 nL of DMSO was transferred to the assay plate as a 0% control (*i.e.,* HC, ZPE) using Echo.

2. Cell seeding

(1) The cell culture medium was discarded, and the cells were washed once with DPBS, digested with trypsin, and counted to prepare a cell suspension of $2\times10^{-5}$ cells/mL.
(2) 25 μL/well of cell suspension was added to the assay plate containing the test compound using Multidrop Combi at medium speed.
(3) The assay plate containing cells was returned to an incubator with 5% $CO_2$ at 37°C and incubated for 16 to 18 hours.

**Day 2**

(1) 25 μL/well of assay reagent (NanoGlo lysis buffer + substrate + LgBit protein) was added to the assay plate using Multidrop Combi at high speed, and the plate was shaken for 10 minutes.
(2) The plate was centrifuged at 2000 rpm for 1 minute to remove air bubbles.
(3) The plate was read on Envision using US Luminescence detection method.

3. Data analysis

The degradation rate (DR) of the test compound was calculated using the following formula: DR (%) = (RLU vehicle control - RLU compound)/(RLU vehicle control - RLU positive control)* 100%, where the vehicle control served as the blank control. The degradation rates of compounds at different concentrations were calculated in Excel, and XLFit software was used to generate inhibition curves and calculate relevant parameters, including minimum degradation rate, maximum degradation rate, and $DC_{50}$.
The test results are shown in Table **9.**

Table **9.** Target protein degradation effect of the compound of formula (I) of the present disclosure in K562 IRAK4-HiBiT cells

| Compound No. | $DC_{50}$ (nM) | Maximum Degradation Rate (%) |
|---|---|---|
| Hydrochloride of Compound of Formula (I) | 4.87 | 108.46 |

**Conclusion:** The compound of the present disclosure exhibits an excellent target protein degradation effect in K562 IRAK4-HiBiT cells.

**Experimental Example 2: In cell western analysis of IKZF1 and IKZF3 protein expression levels of the compound of formula (I) in MM.1S Cells**

**Objective:** The experiment was conducted to evaluate the degradation effects of the compound of formula (I) on IKZF1 and IKZF3 proteins in MM.1S cells by detecting their impact on the expression levels of IKZF1 and IKZF3 proteins in MM.1S cells.

**Experimental materials:**

Cell line: MM.1S cell (obtained from ATCC; Cat. No. CRL-2974)
Negative control: 0.1% DMSO

Table **10.** Reagents and consumables

| Reagents and Consumables | Supplier | Cat. No. |
|---|---|---|
| 96-Well Black Clear-Bottom Plate for ELISA | JingAn Biological | J09603 |
| D-PBS Buffer | Bioleaper | P220610030051 |
| Tissue Fixative Solution | Meilumbio | MA0192-Jan-28G |
| TritonX-100 Solution (10% Sterile) | Beyotime | ST797-500ML |
| Intercept Blocking Buffer | LI-COR | 211114 |
| Ikaros (D6N9Y) Rabbit mAb | Cell Signaling | 14859S |
| Aiolos (D1C1E) Rabbit mAb | Cell Signaling | 15103S |
| GAPDH Mouse mAb | Proteintech | 60004-1-Ig |
| Tween 20 | Sinopharm Chemical Reagent Co., Ltd. | 30189328 |
| IRDye 800CW Goat Anti-Mouse | LI-COR | D00812-08 |
| IRDye 680RD Goat Anti-Rabbit | LI-COR | D00891-05 |

Table **11.** Instruments

| Instrument Name | Supplier | Instrument Model |
|---|---|---|
| Dual-Color Infrared Laser Imager | LI-COR | Odyssey-CLx |
| Decolorization Shaker | QILNBEIER | TS-1000 |
| Refrigerator | Haier Group | BCD-256KT |

**Experimental scheme:**

1) MM.1S cells in the logarithmic growth phase were seeded into a 96-well plate at $1.2 \times 10^5$ cells per well and cultured

overnight.

2) On the second day, drug treatment was initiated with a starting concentration of 300 nM, followed by a 3-fold dilution in triplicate wells, with 10 concentration gradients (containing DMSO), and incubated for 24 hours in a culture incubator.

3) The mixture was centrifuged, and the cell supernatant was carefully removed. Then, 150 μL of 4% paraformaldehyde fixative solution was added along the well wall without touching the bottom cells, followed by incubation at room temperature for 20 min.

4) Permeabilization solution was prepared by adding 0.5 mL of 10% Triton X-100 to 49.5 mL of PBS and mixing thoroughly.

5) 200 μL of the permeabilization solution was added along the well wall without touching the bottom cells, and incubated at room temperature on a shaker for 5 min.

6) The washing step was repeated 4 times.

7) 150 μL of Licor INERCEPT blocking buffer (intertent Blacking buffer) was added along the well wall without touching the bottom cells, and incubated at room temperature on a shaker for 1.5 hours.

8) Ikaros (D6N9Y) Rabbit mAb, Aiolos (D1C1E) Rabbit mAb, and GAPDH Mouse mAb (Proteintech, 60004-1-Ig) were diluted at a ratio of 1:100 using antibody diluent.

9) 50 μL of the mixed antibodies was added to each well in triplicate, and incubated overnight at 4°C on a shaker.

10) PBST (PBS containing 0.1% Tween 20) was prepared.

11) The primary antibody was removed, and 200 μL of PBST was added along the well wall without touching the bottom cells. The mixture was incubated at room temperature on a shaker for 5 min.

12) The washing step was repeated 4 times.

13) The secondary antibody diluent was prepared by adding Tween 20 to the Licor INTERCEPT blocking buffer at a final concentration of 0.2%.

14) The fluorescent secondary antibody was diluted in the dark (1:800 dilution). To 400 μL of the secondary antibody diluent, 0.5 μL each of IRDye 800CW and IRDye 680CW was added (the corresponding fluorescent secondary antibody for the primary antibody).

15) 50 μL of the diluted fluorescent secondary antibody was added to each well, followed by incubation at room temperature on a shaker in the dark for 60 min.

16) The secondary antibody was removed, and 200 μL of PBST was added along the well wall without touching the bottom cells. The plate was then incubated at room temperature on a shaker in the dark for 5 min.

17) The washing step was repeated 4 times. Immediately after washing, the plate was scanned using the Odyssey Gel Imaging System at dual wavelengths of 700 nm and 800 nm.

Data analysis

GraphPad Prism 6 software was utilized to fit the curve with the inhibition rate data, and the $DC_{50}$ value was calculated.

$$\text{Protein inhibition rate} = (1\text{-RLs/RLv})*100\%$$

$$\text{RR (Raw Ratio)} = 700\ \text{nm}/800\ \text{nm}$$

RLs = RR of sample-treated cells
RLv = RR of vehicle-treated cells

The test results are shown in Table **12.**

Table **12.** Degradation effects of the compound of formula (I) of the present disclosure on IKZF1 and IKZF3 proteins in MM.1S cells

| Compound No. | Protein | $DC_{50}$ (nM) | Maximum Degradation Rate Dmax (%) |
|---|---|---|---|
| Hydrochloride of Compound of Formula (I) | IKZF1 | 23.40 | 72.32 |
| | IKZF3 | 20.64 | 73.46 |

**Conclusion:** The compound of the present disclosure exhibits excellent target protein degradation effects on IKZF1 and IKZF3 proteins in MM. 1S cells.

**Experimental Example 3: Evaluation of the anti-proliferative effect of the compound of formula (I) in lymphoma cell lines OCI-LY10 and TMD-8**

**Objective:** This experiment was conducted to detect the inhibitory effects of the compound of formula (I) on cell proliferation in diffuse large B-cell lymphoma cell lines OCI-LY10 and TMD-8.

**Experimental materials:**

Table **13.** Cell lines and culture methods

| Cell Lines | Tumor Type | Growth Characteristics | Culture Media |
|---|---|---|---|
| OCI-LY10 | Lymphoma | Suspension | IMDM + 20% FBS + 55 μM β-Mercaptoethanol |
| TMD-8 | Lymphoma | Suspension | MEM + 10% FBS |

Table **14.** Culture media and reagents

| Culture Media and Reagents | Manufacturer | Cat. No. |
|---|---|---|
| Dulbecco's PBS | Hyclone | SH30256.01 |
| Fetal Bovine Serum (FBS) | GIBCO | 10099-141 |
| Antibiotic-Antimycotic | GIBCO | 15240-062 |
| 0.25% Trypsin | GIBCO | 25200072 |
| DMSO | SIGMA | D2650 |
| β-Mercaptoethanol | SIGMA | 60-24-2 |
| MEM | GIBCO | 11095-080 |
| IMDM | GIBCO | 12440-053 |

1. Multi-well plate

Greiner CELLSTAR® 96-well plate, flat-bottom white (with lid and clear bottom), # 3610.

2. Reagents and instruments for cell viability assay

(1) Promega CellTiter-Glo Luminescent Cell Viability Assay Kit (Promega-G7573).
(2) 2104 EnVision® Plate Reader, PerkinElmer.

**Experimental scheme:**

1. Cell culture

The tumor cell lines were cultured under the above culture conditions in an incubator at 37°C and 5% $CO_2$. The cells were passaged periodically, and those in the logarithmic growth phase were harvested for seeding.

2. Cell seeding

(1) The cells were stained with trypan blue, and the viable cells were counted.
(2) The cell concentration was adjusted to an appropriate concentration.

Table **15.** Cell lines and seeding densities

| Cell Lines | Seeding Densities (cells/well) |
|---|---|
| OCI-LY10 | 5000 |
| TMD-8 | 5000 |

(3) 100 μL of cell suspension was added to each well of the culture plate as shown in the figure above.
(4) The culture plate was incubated overnight in an incubator at 37°C, 5% $CO_2$, and 100% relative humidity.

3. Preparation of compound storage plate

Preparation of stock solution storage plate with 1000 times the initial concentration of the compound: The compound was serially diluted with DMSO from the highest concentration to the lowest concentration. The solution was prepared as needed each time.

4. Preparation of 1000× compound working solution and compound treatment of cells

(1) Preparation of 5× working solution from the initial compound concentration: When performing a 3-fold dilution of the compound, 30 μL of the compound was pipetted from the 1000× stock solution. Subsequently, 20 μL of DMSO was added to the subsequent wells, and 10 μL was sequentially pipetted from the previous concentration to the next. When performing a 5-fold dilution of the compound, 30 μL of the compound was pipetted from the 1000× stock solution, followed by the addition of 24 μL of DMSO to the subsequent wells. Then, 6 μL was sequentially pipetted from the previous concentration to the next. In the vehicle control, 20 μL of DMSO was added. The 1000× compound was diluted 200-fold with culture medium, specifically by adding 1 μL of the 1000× diluted compound to 199 μL of culture medium, followed by pipetting up and down to mix thoroughly.
(2) Drug addition: 25 μL of the 5× compound was added to the cell culture plate.
(3) The 96-well cell plate was returned to the incubator for culturing OCI-LY10 (3-fold or 5-fold dilution, incubated with the drug for 5 days) and TMD-8 (3-fold or 5-fold dilution, incubated with the drug for 5 days).

5. CellTiter-Glo luminescent cell viability assay

The following steps were conducted in accordance with the instructions of the Promega CellTiter-Glo Luminescent Cell Viability Assay Kit (Promega-G7573).

(1) CellTiter-Glo buffer was thawed and brought to room temperature.
(2) CellTiter-Glo substrate was brought to room temperature.
(3) CellTiter-Glo working solution was prepared by adding 10 mL of CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.
(6) 60 μL of CellTiter-Glo working solution was added per well (equal to half the volume of cell culture medium per well). The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected on a 2104 EnVision plate reader.

6. Data analysis

The inhibition rate (IR) of the test compound was calculated using the following formula: IR (%) = (1 - RLU compound/RLU vehicle control)*100%. The inhibition rates of compounds at different concentrations were calculated in Excel, and then GraphPad Prism software was used to plot inhibition curves and calculate relevant parameters, including minimum inhibition rate, maximum inhibition rate, and $IC_{50}$.
The test results are shown in Table **16.**

Table **16.** Inhibitory effects of the compound of formula (I) of the present disclosure on cell proliferation in cell lines OCI-LY10 and TMD-8

| **Compound** No. | OCI-LY10 | | TMD-8 | |
|---|---|---|---|---|
| | $IC_{50}$ (nM) | Maximum Inhibition Rate (%) | $IC_{50}$ (nM) | Maximum Inhibition Rate (%) |
| Hydrochloride of Compound of Formula (I) | 13.66 | 94.54 | 10.34 | 89.86 |
| "/" indicates not detected. | | | | |

**Conclusion:** The compound of the present disclosure exhibits excellent inhibitory effects on cell proliferation in both lymphoma cell lines OCI-LY10 and TMD-8.

**Experimental Example 4: Evaluation of the anti-proliferative effect of the compound of formula (I) in lymphoma cell line SU-DHL-2**

**Objective:** This experiment was conducted to detect the inhibitory effect of the compound of formula (I) on cell proliferation in the lymphoma cell line **SU-DHL-2.**

Table **17.** Cell line and culture method

| Cell Line | Tumor Type | Growth Characteristic | Culture Method |
|---|---|---|---|
| SU-DHL-2 | Lymphoma | Suspension | RPMI1640 + 10% FBS (EXCELL) + 1% Penicillin/Streptomycin |

Table **18.** Culture media and reagents

| Culture Media and Reagents | Manufacturer | Cat. No. |
|---|---|---|
| RPMI 1640 | GIBCO | 22400-089 |
| Dulbecco's PBS | Thermo | SH30028.02B |
| Fetal Bovine Serum (FBS) | Hyclone | 11H233 |
| Antibiotic-Antimycotic | GIBCO | 15240-062 |
| DMSO | SIGMA | D2650 |

1. Multi-well plate

Greiner CELLSTAR 384-well plate, flat-bottom black (with lid), # 781090.

2. Reagents and instruments for cell viability assay

(1) Promega CellTiter-Glo Luminescent Cell Viability Assay Kit (Promega-G7573).
(2) 2104 EnVision® Plate Reader, PerkinElmer.

**Experimental scheme:**

1. Cell culture

The tumor cell lines were cultured under the above culture conditions in an incubator at 37°C and 5% $CO_2$. The cells were passaged periodically, and those in the logarithmic growth phase were harvested for seeding.

2. Cell seeding

(1) The cells were stained with trypan blue, and the viable cells were counted.
(2) The cell concentration was adjusted to an appropriate concentration.

Table **19.** Cell line and seeding density

| **Cell Line** | **Seeding Density (cells/well)** |
|---|---|
| SU-DHL-2 | 1500 |

(3) 50 μL of cell suspension was added to each well of the culture plate as shown in the figure above.
(4) The culture plate was incubated overnight in an incubator at 37°C, 5% $CO_2$, and 100% relative humidity.

3. Preparation of compound storage plate

The drug was administered with an Echo655 instrument. A dosing volume of 50 nL was used, with a final DMSO concentration of 0.1%. The culture plate was centrifuged at 1000 rpm for 1 min and incubated at 37°C, 5% $CO_2$, and 100% relative humidity for 4 days.

4. CellTiter-Glo luminescent cell viability assay

The following steps were conducted in accordance with the instructions of the Promega CellTiter-Glo Luminescent Cell Viability Assay Kit (Promega-G7573).

(1) CellTiter-Glo buffer was thawed and brought to room temperature.
(2) CellTiter-Glo substrate was brought to room temperature.
(3) CellTiter-Glo working solution was prepared by adding CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.
(6) 25 μL (equal to half the volume of cell culture medium in each well) of CellTiter-Glo working solution was added to each well. The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected using a 2104 EnVision Plate Reader.

5. Data analysis

The inhibition rate (IR) of the test compounds was calculated using the following formula: IR (%) = (1 - (RLU compound - RLU blank control)/(RLU vehicle control - RLU blank control))* 100%. The inhibition rates of compounds at different concentrations were calculated in Excel, and then GraphPad Prism software was used to generate inhibition curves and calculate relevant parameters, including the minimum inhibition rate, maximum inhibition rate, and $IC_{50}$.

Table **20.** Inhibitory effect of the compound of formula (I) of the present disclosure on cell proliferation in cell line SU-DHL-2

| Compound No. | SU-DHL-2 $IC_{50}$ (nM) | Maximum Inhibition Rate (%) |
|---|---|---|
| Hydrochloride of Compound of Formula (I) | 28.70 | 93.55 |

**Conclusion:** The compound of the present disclosure exhibits an excellent inhibitory effect on cell proliferation in the lymphoma cell line SU-DHL-2.

**Experimental Example 5: Pharmacokinetic evaluation of the compound of formula (I) in mice**

**Objective:**

C57BL/6 or C57 male mice were selected as the test animals for this study. The LC/MS/MS method was employed to quantitatively determine the plasma drug concentrations at various time points after intravenous injection or intragastric administration of the test compound, aiming to evaluate the pharmacokinetic characteristics of the compound of the present disclosure in mice.

**Experimental materials:**

C57BL/6 or C57 mice (male, 20 to 30g, 7 to 10 weeks old, Beijing Vital River).

**Experimental operation:**

A clear solution of the test compound was injected intravenously into the tail vein of C57BL/6 mice (fasted overnight) (vehicle: 10% DMSO/10% solutol/80% $H_2O$), or administered intragastrically to C57 mice (fed). For intravenous injection, 50 μL of blood was collected via the cheek puncture at 0 h (pre-administration) and 0.083 h, 0.25 h, 0.5 h, 1 h, 2 h, 4 h, 6 h, 8 h, and 24 h after administration, and placed in an anticoagulant tube added with heparin sodium. The mixture was thoroughly vortexed and mixed, and centrifuged at 6000 g for 3 minutes at 2 to 8°C. For intragastric administration, blood was collected via the cheek puncture at 0 h (pre-administration) and 0.083 h, 0.25 h, 0.5 h, 1 h, 2 h, 4 h, 6 h, 8 h, and 24 h after administration, and placed in an anticoagulant tube added with heparin sodium. The mixture was thoroughly vortexed and mixed, and centrifuged at 6000 g for 3 minutes at 2 to 8°C. Plasma drug concentrations were determined by the LC-MS/MS method, and relevant pharmacokinetic parameters were calculated using Phoenix WinNonlin 8.2.0 pharmacokinetic software with non-compartmental model linear-log trapezoidal method.

Table **21.** Pharmacokinetic parameters of the compound of formula (I) of the present disclosure in mice

| Compound No. | Pharmacokinetic Parameters in Mice | | | | | | | |
|---|---|---|---|---|---|---|---|---|
| | Intravenous Injection (2 mg/kg) (fasted) | | | | Intragastric Administration (10 mg/kg) (fed) | | | |
| | Plasma Clearance (mL/min/ kg) | Elimination Half-Life (h) | Apparent Volume of Distribution (L/kg) | Area Under the Drug-Time Curve (0-inf, μM·h) | Peak Concentration (μM) | Time to Peak Concentration (h) | Area Under the Drug-Time Curve (0-inf, μM·h) | Bioavailability F (%) |
| Hydrochloride of Compound of Formula (I) | 7.0 | 4.22 | 1.6 | 5.40 | 0.54 | 1.00 | 4.47 | 18.90 |

"/" indicates not detected.

**Conclusion:** The compound of the present disclosure has high oral systemic plasma exposure ($AUC_{0-inf}$). It has superior pharmacokinetic properties in rodents such as mice.

**Experimental Example 7: Pharmacokinetic evaluation of the compound of formula (I) in beagle dogs**

**Objective:**

Male beagle dogs were selected as the experimental animals in this study. The LC/MS/MS method was employed to quantitatively determine the plasma drug concentrations at different time points after intravenous injection or intragastric administration of the test compound, aiming to evaluate the pharmacokinetic characteristics of the compound of formula (I) of the present disclosure in beagle dogs.

**Experimental materials:**

Beagle dogs (male, 7 to 10 kg, Beijing Mars Biotechnology Co., Ltd.).

**Experimental operation:**

A clear solution of the test compound was slowly injected intravenously into beagle dogs (fed) via the peripheral vein (vehicle: 5% DMSO/10% Solutol/85% $H_2O$), or administered intragastrically to beagle dogs (fed). For intravenous administration, 0.5 mL of blood was collected from the peripheral vein at 0.083, 0.25, 0.5, 1, 2, 4, 6, 8, and 24 h after administration, placed in EDTA-2K anticoagulant tubes, and centrifuged at 3200 g for 10 minutes at 2 to 8°C to separate the supernatant. For intragastric administration, 0.5 mL of blood was collected from the peripheral vein at 0.083, 0.25, 0.5, 1, 2, 4, 6, 8, and 24 h after administration, placed in EDTA-2K anticoagulant tubes, and centrifuged at 3200 g for 10 minutes at 2 to 8°C to separate the supernatant. Plasma drug concentrations were determined by the LC-MS/MS method, and relevant pharmacokinetic parameters were calculated using Phoenix WinNonlin 6.3 pharmacokinetic software with non-compartmental model linear-log trapezoidal method.

The test results are shown in Table **22.**

Table **22.** Pharmacokinetic parameters of the compound of formula (I) of the present disclosure in Beagle dogs

| Compound No. | Pharmacokinetic Parameters in Beagle Dogs | | | | | | | |
|---|---|---|---|---|---|---|---|---|
| | Intravenous Injection (0.5 mg/kg) | | | | Intragastric Administration (5 mg/kg) | | | |
| | Plasma Clearance (mL/min/ kg) | Elimination Half-Life (h) | Appa-rent Distribution Volume (L/kg) | Area Under the Drug-Time Curve (0-inf, μM·h) | Peak Concentration (μM) | Time to Peak Concentration (h) | Area Under the Drug-Time Curve (0-inf, μM·h) | Bioavailability F (%) |
| Hydrochloride of Compound of Formula (I) | 21.3 | 5.03 | 5.83 | 0.44 | 0.41 | 4.00 | 3.20 | 72.6 |

**Conclusion:** The compound of the present disclosure has high oral systemic plasma exposure ($AUC_{0-inf}$). It has superior pharmacokinetic properties in non-rodent animals such as Beagle dogs.

**Experimental Example 8: *In vivo* pharmacodynamic study of the compound of formula (I) in SCID mouse xenograft tumor model of human B-cell lymphoma OCI-LY10 cells**

**Objective:**

The antitumor effect of the compound of formula (I) was evaluated using a SCID mouse xenograft tumor model of human B-cell lymphoma OCI-LY10 cells.

**Experimental materials:**

1. Experimental animals: SCID mice, female, 6 to 8 weeks old, weighing 17 to 20 g. Beijing Vital River Laboratory Animal Technology Co., Ltd.
2. Cell line: The human B-cell lymphoma OCI-LY10 cell line was purchased from Nanjing Cobioer Biosciences Co., Ltd., with catalog number CBP60558.

Table **23.** Reagent information

| Name | Manufacturer | Batch Number | Storage Conditions |
|---|---|---|---|
| IMDM Medium | GIBCO, USA | 2323369 | 4°C |
| Fetal Bovine Serum (FBS) | GIBCO, USA | 2305262RP | -20°C |
| Matrigel (Matrigel) | CORNING | 2062001 | -20°C |

Table **24.** Instruments

| Name | Manufacturer | Model |
|---|---|---|
| SHJ Series Clean Bench | Shanghai Shangjing Purification Equipment Co., Ltd. | CA-1390-1 |
| $CO_2$ Water-Jacketed Cell Culture Incubator | Thermo Scientific Forma | 3111 |
| Inverted Microscope | Olympus | CKX41SF |
| Electric Suction Device | Shanghai Medical Instruments (Group) Co., Ltd. | YX930D |
| Eppendorf AG Centrifuge | Eppendorf | 5811XG639987 |
| Balance (0.01 g Precision) | Shanghai Minqiao Precise Science Instrument | SL502N |
| | Co., Ltd. | |
| Balance (0.001 g Precision) | Shanghai Jinghai Instrument Co., Ltd. | JA2003N |
| Electronic Digital Caliper | SHAHE | (0 to 150) mm |

**Model establishment:**

OCI-LY10 cells were cultured in IMDM medium containing 20% FBS and maintained in a 5% $CO_2$ incubator at 37°C with saturated humidity. OCI-LY10 cells in the logarithmic growth phase were collected, resuspended in IMDM basal medium, mixed 1:1 with Matrigel, and adjusted to a cell concentration of $4\times10^7$/mL. Under sterile conditions, 0.1 mL of the cell suspension was subcutaneously inoculated into the right back of SCID mice at an inoculation concentration of $4\times10^6$/0.1 mL/mouse.

**Experimental scheme:**

In pharmacodynamic experiments, when the tumors reached a certain size, animals with excessively large or small tumor volumes or irregular tumor shapes were excluded. Animals with tumor volumes ranging from 167.65 to 231.29 $mm^3$ were selected and randomly grouped based on tumor volume, with 6 mice per group. The average tumor volume was approximately 201.15 $mm^3$. The day of grouping was designated as Day 0, and drug administration was initiated according to the animals' body weights. The pharmacodynamic experiment period was 28 days, with the drug administered once a day with a dosing interval of 24 hours, and administered intragastrically. During the experiment, animal body weight and tumor size were measured twice weekly. Clinical symptoms were observed and recorded daily.

The test compounds were administered at doses of 10 mg/kg, 30 mg/kg, and 100 mg/kg, with the vehicle being 10% DMSO/10% Solutol/80% $H_2O$. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group)/(average tumor volume at the end of treatment in the vehicle control group - average tumor volume at the beginning of treatment in the vehicle control group)]$\times$100%. The relative tumor proliferation rate (T/C, %) was calculated using the formula: T/C % = $T_{RTV}/C_{RTV} \times 100\%$ ($T_{RTV}$: mean RTV of the treatment group; $C_{RTV}$: mean RTV of the negative control group). The relative tumor volume (RTV) was calculated based on the tumor measurement results, with the formula RTV = $V_t/V_0$, where $V_0$ is the tumor volume measured at the time of grouping administration (*i.e.*, Day 0), and $V_t$ is the tumor volume at a certain measurement time. $T_{RTV}$ and $C_{RTV}$ were derived from data on the same day.

**Data analysis:**

In this study, experimental data were expressed as Mean$\pm$SEM.

Statistical analysis was performed using IBM SPSS Statistics software based on RTV data at the end of the experiment. Comparisons between two groups were analyzed by T test, while comparisons among three or more groups were analyzed by one-way ANOVA. If the variance was homogeneous (no significant difference in F-value), Tukey's method was applied for analysis. If the variance was heterogeneous (significant difference in F-value), the Games-Howell method was applied for testing. $p < 0.05$ was considered to be significantly different.

**Experimental results:** Test results are shown in Tables **25** and **26.**

Table **25.** Evaluation of the tumor-inhibitory efficacy of the compound of formula (I) of the present disclosure in a subcutaneous xenograft tumor model of human B-cell lymphoma OCI-LY10 cells

| Group | Dosage | Tumor Volume ($mm^3$)[a] (Day 0) | Tumor Volume ($mm^3$)[a] (Day 28) | Relative Tumor Volume[a] (Day 28) | T/C (%) (Day 28) | TGI (%) (Day 28) |
|---|---|---|---|---|---|---|
| Vehicle Control | 0 mg/kg | 201.76 ± 7.08 | 1422.84 ± 89.14 | 7.11 ± 0.56 | -- | -- |
| Hydrochloride of Compound of Formula (I) | 10 mg/kg | 202.43 ± 8.41 | 685.61 ± 75.95 | 3.37 ± 0.34 | 47.40 | 60.43 |
| | 30 mg/kg | 199.80 ± 8.02 | 322.68 ± 20.47 | 1.64 ± 0.17 | 23.07 | 89.94 |
| | 100 mg/kg | 199.75 ± 7.96 | 188.01 ± 35.59 | 0.91 ± 0.15 | 12.80 | 100.96 |
| Note: a. Mean ± SEM. | | | | | | |

Table **26.** *p* values for comparison of relative tumor volumes among groups in the human B-cell lymphoma OCI-LY10 xenograft tumor model treated with the compound of the present disclosure

| Group/Dosage | Vehicle Control | Hydrochloride of Compound of Formula (I) | | |
|---|---|---|---|---|
| | | 10 mg/kg | 30 mg/kg | 100 mg/kg |
| Vehicle Control | N/A | 0.003 | 0.001 | <0.001 |

(continued)

| Group/Dosage | | Vehicle Control | Hydrochloride of Compound of Formula (I) | | |
|---|---|---|---|---|---|
| | | | 10 mg/kg | 30 mg/kg | 100 mg/kg |
| Hydrochloride of Compound of Formula (I) | 10 mg/kg | 0.003 | N/A | 0.018 | 0.003 |
| | 30 mg/kg | 0.001 | 0.018 | N/A | 0.077 |
| | 100 mg/kg | <0.001 | 0.003 | 0.077 | N/A |
| Note: The *p* value was analyzed using IBM SPSS Statistics software. | | | | | |

**Conclusion:** The compound of the present disclosure exhibits a significant tumor inhibitory effect in the SCID mouse xenograft tumor model of human B-cell lymphoma OCI-LY10 cells in a dose-dependent manner.

**Experimental Example 9: *In vivo* pharmacodynamic study of the compound of formula (I) in the CB17 SCID mouse subcutaneous xenograft tumor model of human lymphoma SU-DHL-2 cells**

**Objective:**

The antitumor effect of the compound of formula (I) was evaluated using a SU-DHL-2 subcutaneous xenograft tumor model of CB17 SCID mice.

**Experimental materials:**

1. Experimental animals: CB17 SCID mice, female, 6 to 8 weeks old, weighing 18 to 22 g. Beijing Vital River Laboratory Animal Technology Co., Ltd.
2. Cell line: Human lymphoma SU-DHL-2 cells (Cat. No.: ATCC-CRL-2956).

Table **27.** Reagent information

| Name | Manufacturer | Cat. No. | Batch Number | Expiration Date | Storage Conditions |
|---|---|---|---|---|---|
| 1640 Medium | Gibco | 22400089 | 2462009 | 2023-02-28 | 4°C |
| Matrigel | Corning | 354234 | 2013002 | 2022-10-19 | -20°C |
| 100× Double Antibiotics (Penicillin, Streptomycin) | Meilunbio | MA0110 | MA0110-Apr-15H | 2023-04-14 | -20°C |
| PBS | HyClone | SH30256.01 | AG29791799 | 2023-08-31 | 4°C |
| Fetal Bovine Serum | ExCell Bio | FND500 | 11H233 | 2023-08 | -20°C |

Table **28.** Instruments

| Name | Manufacturer | Model |
|---|---|---|
| Biosafety Cabinet | Suzhou Antai Airtech Co., Ltd. | BSC-160411A2 |
| Vernier Caliper | Mitutoyo | CD-6"ASX |
| Electronic Balance | Changzhou Tianzhiping Instruments Co., Ltd. | EL-2KJ |
| Vortex Vibrator | HaiMen City Qilin Medical Instrument Factory | XW-80A |
| Pure Water System | Millipore | MILLI-Q®Direct8 |

**Model establishment:**

Cell culture: Human lymphoma SU-DHL-2 cells (ATCC-CRL-2956) were cultured in suspension *in vitro* under conditions of RPMI 1640 medium supplemented with 10% inactivated fetal bovine serum, 100 U/mL penicillin, and 100 μg/mL streptomycin, and incubated at 37°C in a 5% $CO_2$ incubator. Routine passaging was performed twice a week. When the cell saturation density was 80% to 90% and the number of cells reached the required level, the cells were collected, counted, and inoculated.

Tumor cell inoculation and grouping: 0.2 mL ($10\times10^6$ cells) (PBS: matrigel = 1:1) of SU-DHL-2 cells were subcutaneously inoculated into the right back of each mouse. When the average tumor volume reached approximately 139 $mm^3$, the drugs were administered in groups. The day of grouping was designated as Day 0, and drug administration was initiated according to the animals' body weights.

**Experimental scheme:**

The pharmacodynamic experiment was conducted with a 7-day administration cycle, where the test compound was administered once daily intragastrically at 24-hour intervals, for a total of three cycles. During the experiment, animal body weight and tumor size were measured twice a week, and clinical symptoms were observed and recorded daily.

The test compounds were administered at doses of 10 mg/kg, 30 mg/kg, and 100 mg/kg, with the vehicle being 10% DMSO/10% Solutol/80% water. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group))/(average tumor volume at the end of treatment in the vehicle control group - average tumor volume at the beginning of treatment in the vehicle control group)] × 100%. The relative tumor proliferation rate (T/C, %) was calculated using the formula: T/C % = $T_{RTV}/C_{RTV}\times100\%$ ($T_{RTV}$: mean RTV of the treatment group; $C_{RTV}$: mean RTV of the negative control group). The relative tumor volume (RTV) was calculated based on the tumor measurement results, with the formula RTV = $V_1/V_0$, where $V_0$ is the tumor volume measured at the time of grouping administration (*i.e.,* Day 0), and $V_t$ is the tumor volume at a certain measurement time. $T_{RTV}$ and $C_{RTV}$ were derived from data on the same day.

Data analysis:

Statistical analysis was performed using SPSS software based on RTV data at the end of the experiment. Comparisons between two groups were analyzed by T test, while comparisons among three or more groups were analyzed by one-way ANOVA. If the variance was homogeneous (no significant difference in F-value), Tukey's method was applied for analysis. If the variance was heterogeneous (significant difference in F-value), the Games-Howell method was applied for testing. $p < 0.05$ was considered to be significantly different.

**Experimental results:** Test results are shown in Tables **29** and **30.**

Table **29.** Evaluation of the tumor-inhibitory efficacy of the compound of formula (I) of the present disclosure in the subcutaneous xenograft tumor model of lymphoma SU-DHL-2 cells

| Group | Dosage | Tumor Volume ($mm^3$)[a] (Day 0) | Tumor Volume ($mm^3$)[a] (Day 21) | Relative Tumor Volume[a] (Day 21) | T/C (%) (Day 21) | TGI (%) (Day 21) |
|---|---|---|---|---|---|---|
| Vehicle Control | 0 mg/kg | 139 ± 6 | 1883 ± 141 | 13.68 | - | - |
| Hydrochloride of Compound of Formula (I) | 10 mg/kg | 139 ± 6 | 800 ± 48 | 5.79 | 42.33 | 62.13 |
| | 30 mg/kg | 139 ± 6 | 813 ± 99 | 5.78 | 42.26 | 61.41 |
| | 100 mg/kg | 139 ± 5 | 626 ± 51 | 4.55 | 33.23 | 72.07 |
| Note: a. Mean ± SEM. | | | | | | |

Table **30**. *p* values for comparison of relative tumor volumes among groups in the lymphoma SU-DHL-2 xenograft tumor model treated with the compound of formula (I) of the present disclosure

| Group/Dosage | | Vehicle Control | Hydrochloride of Compound of Formula (I) | | |
|---|---|---|---|---|---|
| | | | 10 mg/kg | 30 mg/kg | 100 mg/kg |
| Vehicle Control | | N/A | 0.001 | 0.001 | <0.001 |
| Hydrochloride of Compound of Formula (I) | 10 mg/kg | 0.001 | N/A | 1.000 | 0.268 |
| | 30 mg/kg | 0.001 | 1.000 | N/A | 0.507 |
| | 100 mg/kg | <0.001 | 0.268 | 0.507 | N/A |
| Note: The *p* value was obtained by analyzing the relative tumor volume (RTV) using one-way ANOVA. Data with heterogeneous variance were analyzed using Games-Howell. | | | | | |

**Conclusion:** The compound of the present disclosure exhibits a significant tumor inhibitory effect in a CB17 SCID mouse subcutaneous xenograft tumor model of human lymphoma SU-DHL-2 cells.

**Experimental Example 10: *In vivo* pharmacodynamic study of the compound of formula (I) on a BALB/c nude mouse subcutaneous xenograft tumor model of human diffuse large B-cell lymphoma TMD-8 cells**

**Objective:**

The antitumor effect of the compound of formula (I) was evaluated using a BALB/c nude mouse subcutaneous xenograft tumor model of human diffuse large B-cell lymphoma TMD-8 cells.

**Experimental materials:**

1. Experimental animals: BALB/c nude mice, female, 6 to 8 weeks old. Vital River Laboratory Animal Technology Co., Ltd.
2. Cell line: Human diffuse large B-cell lymphoma TMD-8 cells (purchased from Shanghai Huzhen Industrial Co., Ltd.).

Table **31.** Main reagent information

| Name | Manufacturer | Cat. No. | Batch Number |
|---|---|---|---|
| RPMI-1640 Powder | Gibco | 31800-022 | 2383858 |
| Fetal Bovine Serum (FBS) | Gibco | 10099-141C | 2217479CP |
| Matrigel | Corning | 354248 | 2139002 |
| PBS | Beyotime | ST447 | 100921220308 |
| Double Antibiotics | Hyclone | SV30010 | J210026 |
| DMSO | Aladdin | D103273 | H2120209 |
| Solutol HS15 | BASF | 50379938 | 42608009T0 |
| Sterile Water for Injection | Zhejiang Dubang Pharmaceutical | NA | 2111290103 |

Table **32.** Main instrument information

| Name | Manufacturer | Model |
|---|---|---|
| Vernier Caliper | Mitutoyo Corporation | CD-6"CX |
| Ultrasonic Homogenizer | Kunshan Ultrasonic Instruments Co., Ltd. | KQ-700DE |
| Balance | METTLER TOLEDO | XP 105 |

(continued)

| Name | Manufacturer | Model |
|---|---|---|
| Vortex Mixer | Thermolyne | 16700 |

**Model establishment:**

Cell culture: Routine cell culture was conducted in RPMI-1640 medium containing 10% fetal bovine serum under 5% $CO_2$ at 37°C. Cells were passaged based on cell growth conditions at a passage ratio of 1:3 to 1:4.

Tumor cell inoculation and grouping: TMD-8 cells in the logarithmic growth phase were harvested, counted, and resuspended in a mixture of 50% serum-free RPMI-1640 medium and 50% Matrigel. The cell concentration was adjusted to $4.0\times10^7$ cells/mL. The cell suspension was placed in an ice box, aspirated using a 1-mL syringe, and injected subcutaneously into the right forelimb axilla of nude mice at 200 μL per animal ($0.8\times10^7$ cells/mouse) to establish the TMD-8 xenograft tumor model. Administration was started when the average tumor volume reached approximately 160 $mm^3$. The day of grouping was designated as Day 1 (D1) of the experiment, and administration was started based on animal body weight.

**Experimental scheme:**

The pharmacodynamic experiment was conducted with a 7-day administration cycle, where the test compound was administered once daily intragastrically at 24-hour intervals, for a total of three cycles. During the experiment, animal body weight and tumor size were measured twice a week, and clinical symptoms were observed and recorded daily.

The test compound was administered at doses of 10 mg/kg, 30 mg/kg, and 100 mg/kg, with the vehicle being 10% DMSO/10% Solutol HS15/80% water. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group)/(average tumor volume at the end of treatment in the vehicle control group - average tumor volume at the beginning of treatment in the vehicle control group)]×100%. The relative tumor proliferation rate (T/C, %) was calculated using the formula: T/C % = $T_{RTV}/C_{RTV}\times100\%$ ($T_{RTV}$: mean RTV of the treatment group; $C_{RTV}$: mean RTV of the negative control group). The relative tumor volume (RTV) was calculated based on the tumor measurement results, with the formula RTV = $V_t/V_0$, where $V_0$ is the tumor volume measured at the time of grouping administration (*i.e.*, Day 1), and $V_t$ is the tumor volume at a certain measurement time. $T_{RTV}$ and $C_{RTV}$ were derived from data on the same day.

**Data analysis:**

The experimental data were calculated and subjected to relevant statistical processing using Microsoft Office Excel 2007 software. Unless otherwise specified, the data were expressed as mean ± standard error (Mean ± SE), and comparisons between groups were performed using t-tests.

**Experimental results:** The test results are shown in Table **33.**

Table **33.** Evaluation of the tumor-inhibitory efficacy of the compound of formula (I) of the present disclosure in the nude mouse xenograft tumor model of human diffuse large B-cell lymphoma TMD-8 cells

| Group | Dosage | Tumor Volume ($mm^3$)[a] (Day 0) | Tumor Volume ($mm^3$)[a] (Day 22) | Relative Tumor Volume[a] (Day 22) | T/C (%) (Day 22) | TGI (%) (Day 22) |
|---|---|---|---|---|---|---|
| Vehicle Control | 0 mg/kg | 160 ± 10 | 1234 ± 179 | 7.62 | - | - |

(continued)

| Group | Dosage | Tumor Volume ($mm^3$)[a] (Day 0) | Tumor Volume ($mm^3$)[a] (Day 22) | Relative Tumor Volume[a] (Day 22) | T/C (%) (Day 22) | TGI (%) (Day 22) |
|---|---|---|---|---|---|---|
| Hydrochloride of Compound of Formula (I) | 10 mg/kg | 161 ± 12 | 714 ± 153* | 4.41 | 58 | 49 |
| | 30 mg/kg | 160 ± 9 | 430 ± 98** | 2.60 | 34 | 75 |
| | 100 mg/kg | 160 ± 8 | 291 ± 70** | 1.78 | 23 | 88 |
| Note: "*" indicates a significant difference in tumor volume compared to the vehicle control group ($P$ < 0.05); "**" indicates a highly significant difference compared to the vehicle control group ($P$ < 0.01); "a" indicates mean ± SEM. | | | | | | |

**Conclusion:** The compound of the present disclosure exhibits a significant tumor inhibitory effect in the BALB/c nude mouse subcutaneous xenograft tumor model of human diffuse large B-cell lymphoma TMD-8 cells.

**Claims**

1. A compound of formula (II) or a crystal form thereof,

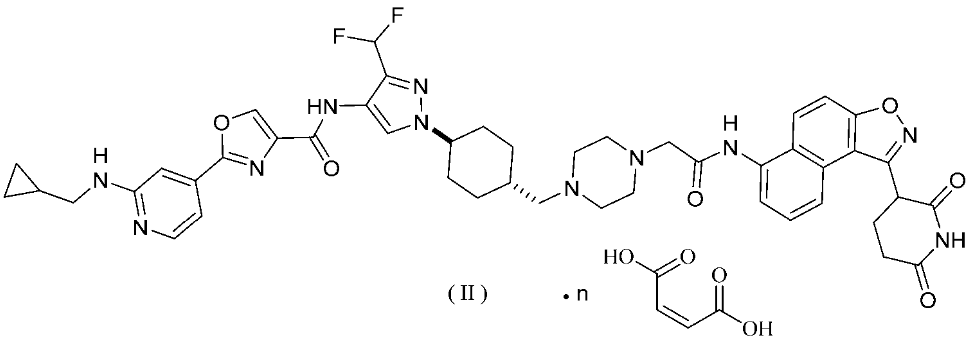

(II) • n

,

wherein n is 1.9 to 2.1; preferably 1.9, 2.0, or 2.1.

2. The crystal form of the compound of formula (II) according to claim 1, wherein the crystal form of the compound of formula (II) has an X-ray powder diffraction pattern comprising characteristic diffraction peaks at the following 2θ angles: 6.67±0.20°, 9.52±0.20°, 17.03±0.20°, and 19.31±0.20°.

3. The crystal form of the compound of formula (II) according to claim 2, wherein the X-ray powder diffraction pattern thereof comprises characteristic diffraction peaks at the following 2θ angles: 6.67°, 9.52°, 17.03°, and 19.31°.

4. The crystal form of the compound of formula (II) according to claim 2, wherein the X-ray powder diffraction pattern thereof is as shown in FIG. 1.

5. The crystal form of the compound of formula (II) according to claim 2, wherein the crystal form of the compound of formula (II) has a differential scanning calorimetry curve showing endothermic peaks with peak temperatures at 153.3°C and 172.3°C.

6. The crystal form of the compound of formula (II) according to claim 2, wherein the crystal form of the compound of formula (II) has a differential scanning calorimetry pattern as shown in FIG. 2.

7. The crystal form of the compound of formula (II) according to claim 2, wherein the crystal form of the compound of formula (II) has a thermogravimetric analysis curve showing a weight loss of 6.42% at 150.0°C.

8. The crystal form of the compound of formula (II) according to claim 2, wherein the crystal form of the compound of formula (II) has a thermogravimetric analysis pattern as shown in FIG. 3.

**9.** A compound of formula (III) or a crystal form thereof,

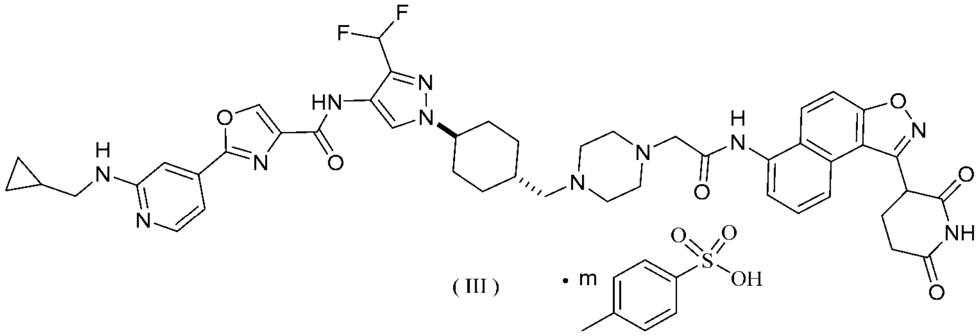

(III)

,

wherein m is 1.1 to 1.5; preferably 1.1, 1.2, 1.3, 1.4, or 1.5.

**10.** The crystal form of the compound of formula (III) according to claim 9, wherein the crystal form of the compound of formula (III) has an X-ray powder diffraction pattern comprising characteristic diffraction peaks at the following 2θ angles: 10.61±0.20°, 12.06±0.20°, 15.79±0.20°, and 17.96±0.20°.

**11.** The crystal form of the compound of formula (III) according to claim 9, wherein the X-ray powder diffraction pattern thereof comprises characteristic diffraction peaks at the following 2θ angles: 10.61°, 12.06°, 15.79°, and 17.96°.

**12.** The crystal form of the compound of formula (III) according to claim 9, wherein the X-ray powder diffraction pattern thereof is as shown in FIG. 4.

**13.** The crystal form of the compound of formula (III) according to claim 9, wherein the crystal form of the compound of formula (III) has a differential scanning calorimetry curve showing endothermic peaks with peak temperatures at 78.5°C, 158.7°C, and 169.0°C.

**14.** The crystal form of the compound of formula (III) according to claim 9, wherein the crystal form of the compound of formula (III) has a differential scanning calorimetry pattern as shown in FIG. 5.

**15.** The crystal form of the compound of formula (III) according to claim 9, wherein the crystal form of the compound of formula (III) has a thermogravimetric analysis curve showing a weight loss of 5.16% at 150.0°C.

**16.** The crystal form of the compound of formula (III) according to claim 9, wherein the crystal form of the compound of formula (III) has a thermogravimetric analysis pattern as shown in FIG. 6.

**17.** A compound of formula (IV) or a crystal form thereof,

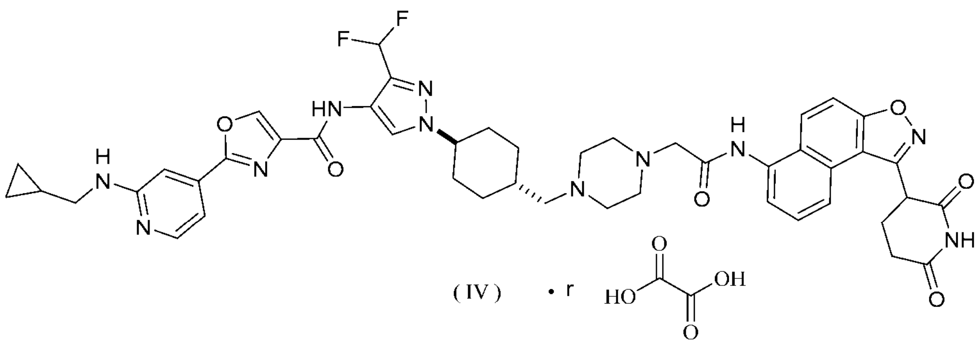

(IV)

,

wherein r is 2.1 to 2.6; preferably 2.1, 2.2, 2.3, 2.4, 2.5, or 2.6.

**18.** The crystal form of the compound of formula (IV) according to claim 17, wherein the crystal form of the compound of formula (IV) has an X-ray powder diffraction pattern comprising characteristic diffraction peaks at the following 2θ

angles: 5.65±0.20°, 11.16±0.20°, and 19.49±0.20°.

**19.** The crystal form of the compound of formula (IV) according to claim 18, wherein the X-ray powder diffraction pattern thereof comprises characteristic diffraction peaks at the following 2θ angles: 5.65±0.20°, 8.39±0.20°, 11.16±0.20°, 17.03±0.20°, 19.49±0.20°, 22.26±0.20°, and 23.09±0.20°.

**20.** The crystal form of the compound of formula (IV) according to claim 19, wherein the X-ray powder diffraction pattern thereof comprises characteristic diffraction peaks at the following 2θ angles: 5.65°, 8.39°, 11.16°, 17.03°, 19.49°, 22.26°, and 23.09°.

**21.** The crystal form of the compound of formula (IV) according to claim 19, wherein the X-ray powder diffraction pattern thereof is as shown in FIG. 7.

**22.** The crystal form of the compound of formula (IV) according to claim 19, wherein the crystal form of the compound of formula (IV) has a differential scanning calorimetry curve showing an endothermic peak with a peak temperature at 216.9°C.

**23.** The crystal form of the compound of formula (IV) according to claim 19, wherein the crystal form of the compound of formula (IV) has a differential scanning calorimetry pattern as shown in FIG. 8.

**24.** The crystal form of the compound of formula (IV) according to claim 19, wherein the crystal form of the compound of formula (IV) has a thermogravimetric analysis curve showing a weight loss of 6.40% at 150.0°C.

**25.** The crystal form of the compound of formula (IV) according to claim 19, wherein the crystal form of the compound of formula (IV) has a thermogravimetric analysis pattern as shown in FIG. 9.

**26.** A compound of formula (V) or a crystal form thereof,

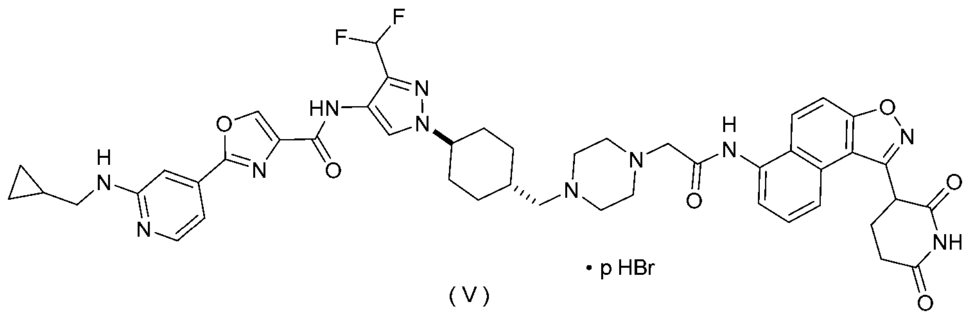

• p HBr

(V)

,

wherein p is 1.0 to 1.5; preferably 1.0, 1.1, 1.2, 1.3, 1.4, or 1.5.

**27.** The crystal form of the compound of formula (V) according to claim 26, wherein the crystal form of the compound of formula (V) has an X-ray powder diffraction pattern comprising characteristic diffraction peaks at the following 2θ angles: 6.51±0.20°, 8.94±0.20°, 17.87±0.20°, 19.88±0.20°, and 21.51±0.20°.

**28.** The crystal form of the compound of formula (V) according to claim 27, wherein the X-ray powder diffraction pattern thereof comprises characteristic diffraction peaks at the following 2θ angles: 6.51±0.20°, 8.94±0.20°, 9.76±0.20°, 13.09±0.20°, 17.87±0.20°, 19.88±0.20°, 21.51±0.20°, and 27.11±0.20°.

**29.** The crystal form of the compound of formula (V) according to claim 28, wherein the X-ray powder diffraction pattern thereof comprises characteristic diffraction peaks at the following 2θ angles: 6.51°, 8.94°, 9.76°, 13.09°, 17.87°, 19.88°, 21.51°, 27.11°, and 29.37°.

**30.** The crystal form of the compound of formula (V) according to claim 29, wherein the X-ray powder diffraction pattern thereof is as shown in FIG. 10.

**31.** The crystal form of the compound of formula (V) according to claim 29, wherein the crystal form of the compound of

formula (V) has a differential scanning calorimetry curve showing endothermic peaks with peak temperatures at 64.7°C and 217.5°C.

**32.** The crystal form of the compound of formula (V) according to claim 29, wherein the crystal form of the compound of formula (V) has a differential scanning calorimetry pattern as shown in FIG. 11.

**33.** The crystal form of the compound of formula (V) according to claim 29, wherein the crystal form of the compound of formula (V) has a thermogravimetric analysis curve showing a weight loss of 4.06% at 150.0°C.

**34.** The crystal form of the compound of formula (V) according to claim 29, wherein the crystal form of the compound of formula (V) has a thermogravimetric analysis pattern as shown in FIG. 12.

**35.** A compound of formula (VI) or a crystal form thereof,

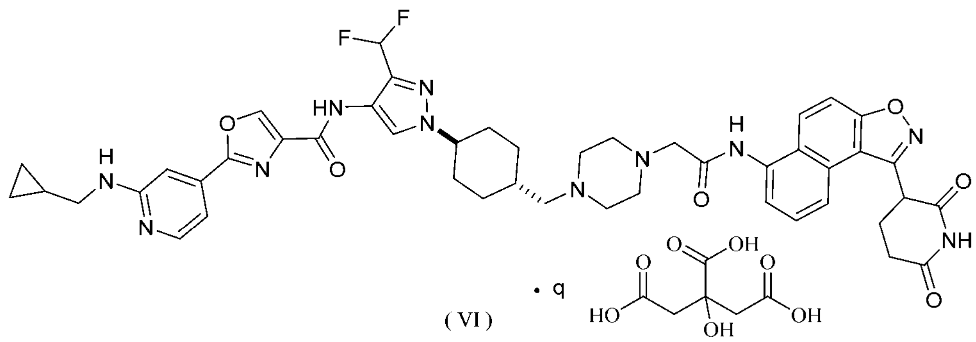

(VI)

,

wherein q is 2.1 to 2.3; preferably 2.1, 2.2, or 2.3.

**36.** The crystal form of the compound of formula (VI) according to claim 35, wherein the crystal form of the compound of formula (VI) has an X-ray powder diffraction pattern comprising characteristic diffraction peaks at the following 2θ angles: 7.63±0.20° and 18.46±0.20°.

**37.** The crystal form of the compound of formula (VI) according to claim 35, wherein the X-ray powder diffraction pattern thereof comprises characteristic diffraction peaks at the following 20 angles: 7.63° and 18.46°.

**38.** The crystal form of the compound of formula (VI) according to claim 35, wherein the X-ray powder diffraction pattern thereof is as shown in FIG. 13.

**39.** The crystal form of the compound of formula (VI) according to claim 35, wherein the crystal form of the compound of formula (VI) has a differential scanning calorimetry curve showing endothermic peaks with peak temperatures at 77.6°C, 159.4°C, and 184.2°C.

**40.** The crystal form of the compound of formula (VI) according to claim 35, wherein the crystal form of the compound of formula (VI) has a differential scanning calorimetry pattern as shown in FIG. 14.

**41.** The crystal form of the compound of formula (VI) according to claim 35, wherein the crystal form of the compound of formula (VI) has a thermogravimetric analysis curve showing a weight loss of 7.05% at 150.0°C.

**42.** The crystal form of the compound of formula (VI) according to claim 35, wherein the crystal form of the compound of formula (VI) has a thermogravimetric analysis pattern as shown in FIG. 15.

**43.** Use of the compound of formula (II) or the crystal form thereof according to claim 1, the crystal form of the compound of formula (II) according to any one of claims 2 to 8, the compound of formula (III) or the crystal form thereof according to claim 9, the crystal form of the compound of formula (III) according to any one of claims 10 to 16, the compound of formula (IV) or the crystal form thereof according to claim 17, the crystal form of the compound of formula (IV) according to any one of claims 18 to 25, the compound of formula (V) or the crystal form thereof according to claim 26, the crystal form of the compound of formula (V) according to any one of claims 27 to 34, the compound of formula (VI) or the crystal form thereof according to claim 35, or the crystal form of the compound of formula (VI) according to any one

of claims 36 to 42 in the manufacture of a medicament for treating diffuse large B-cell lymphoma.

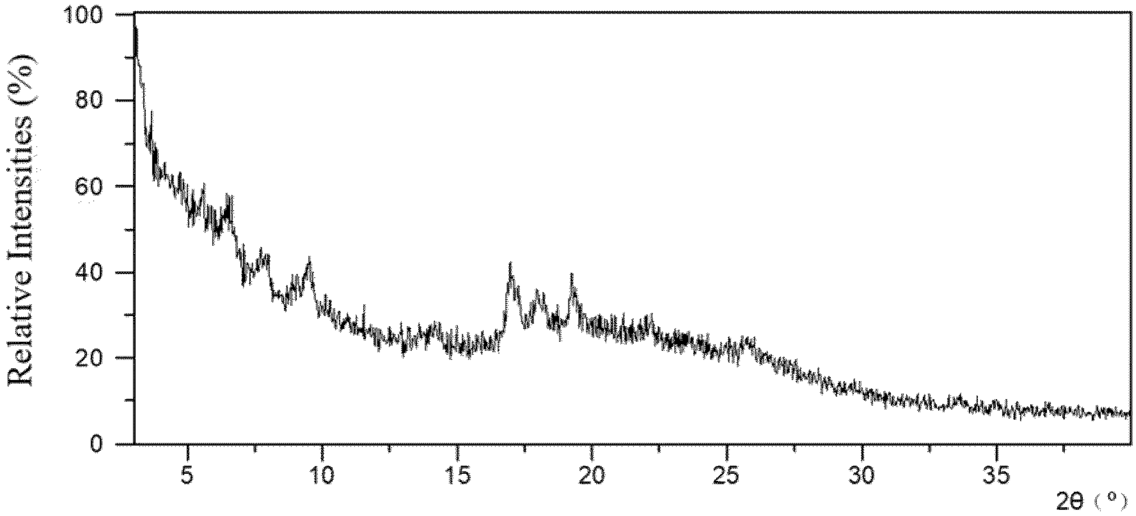
100
80
60
40
20
0
Relative Intensities (%)
5
10
15
20
25
30
35
2θ (°)

FIG. 1

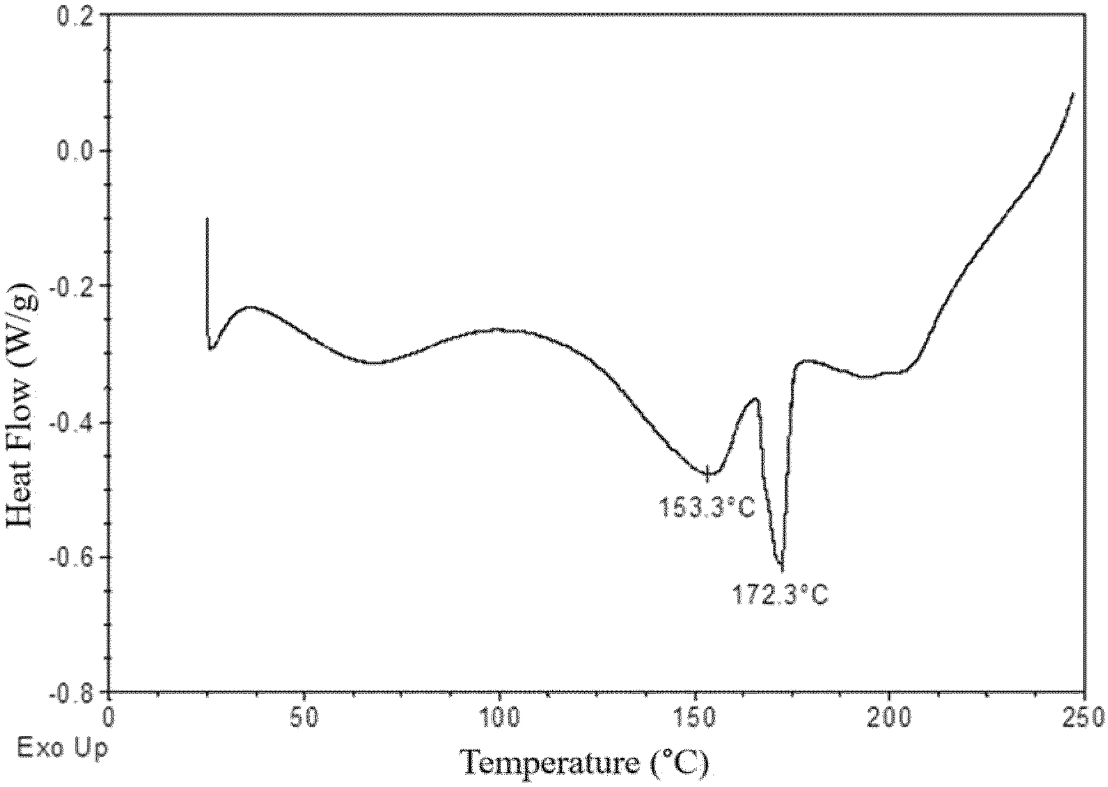
0.2
0.0
-0.2
-0.4
-0.6
-0.8
Heat Flow (W/g)
153.3°C
172.3°C
0
50
100
150
200
250
Exo Up
Temperature (°C)

FIG. 2

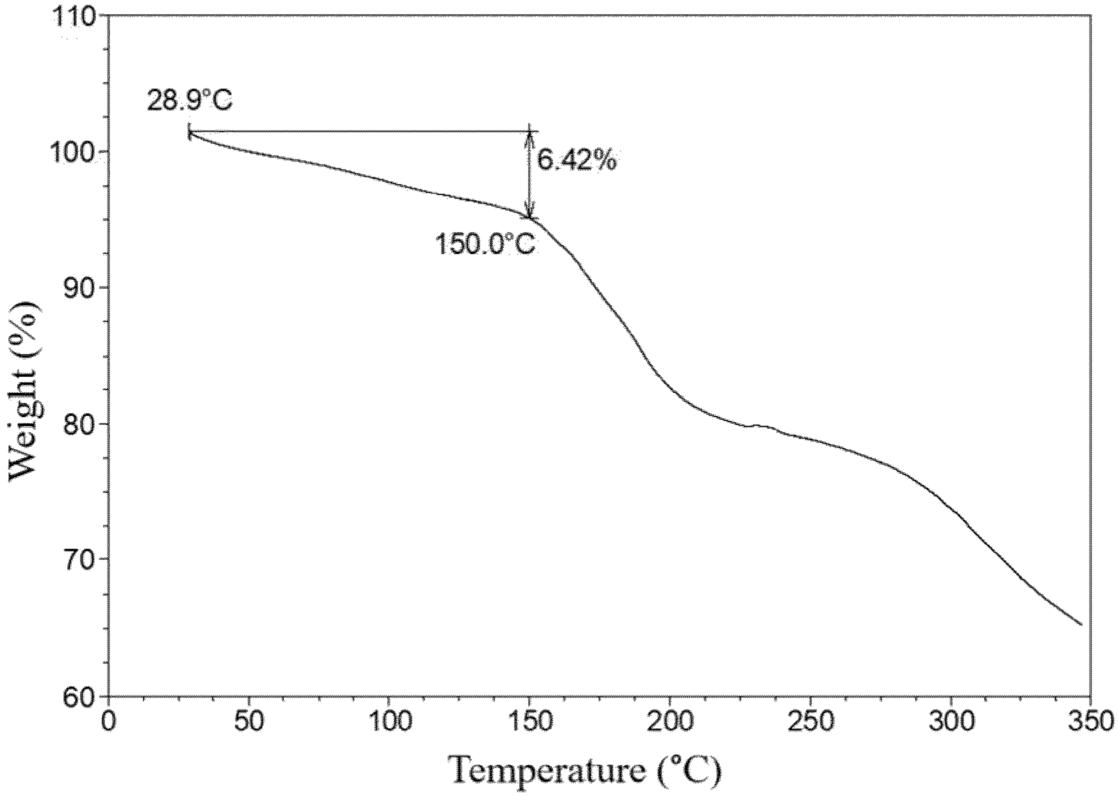
110
100
90
80
70
60
0
50
100
150
200
250
300
350
28.9°C
6.42%
150.0°C
Weight (%)
Temperature (°C)

FIG. 3

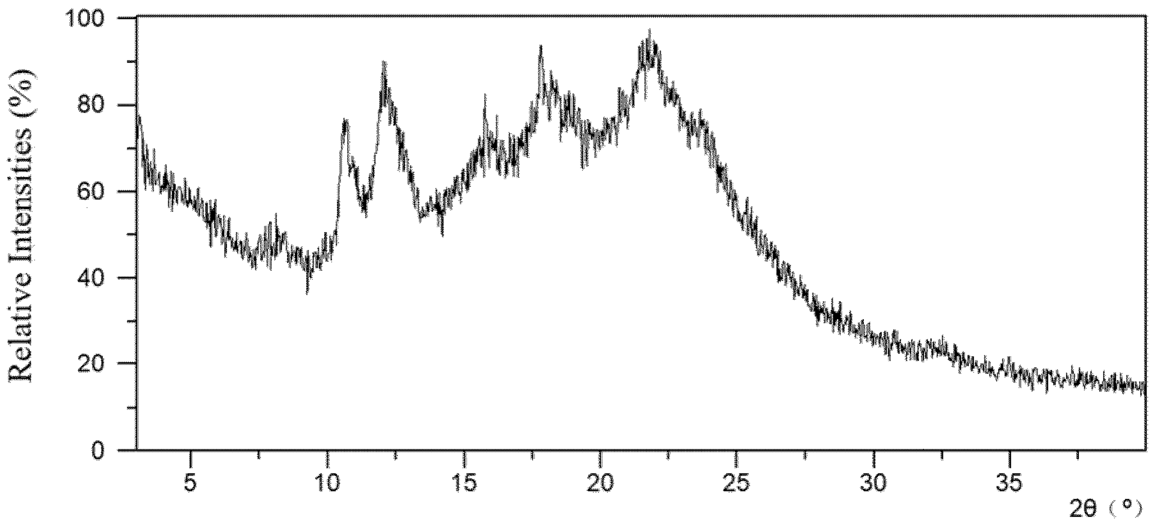
100
80
60
40
20
0
5
10
15
20
25
30
35
2θ（°）
Relative Intensities (%)

FIG. 4

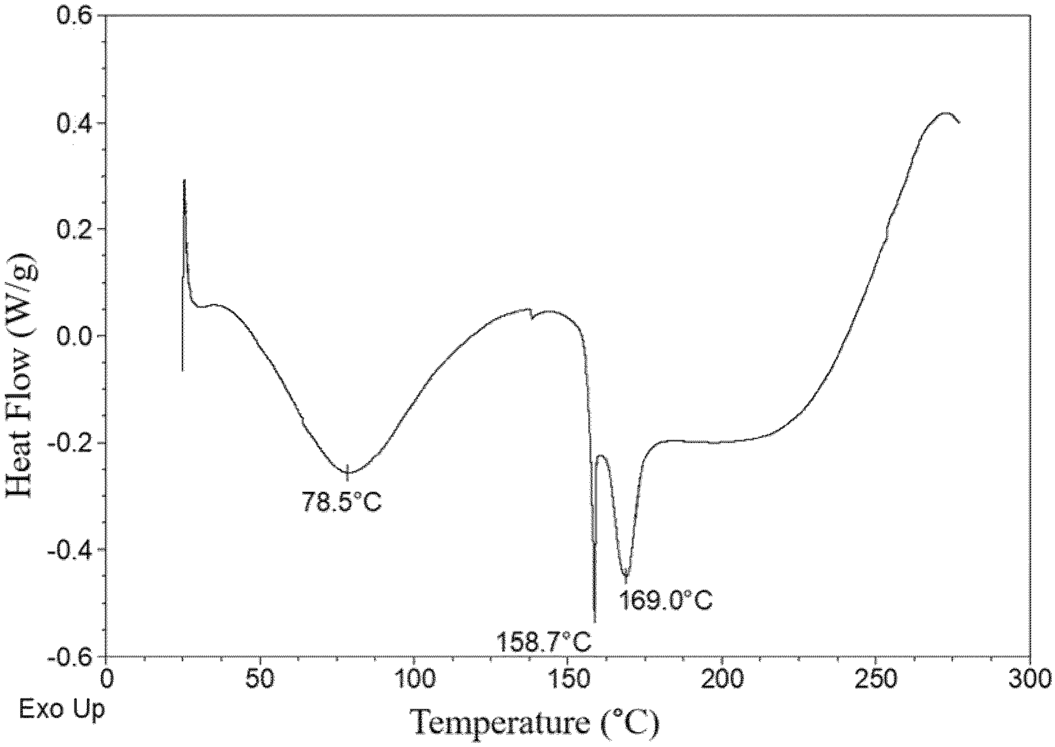
0.6
0.4
0.2
0.0
-0.2
-0.4
-0.6
Heat Flow (W/g)
78.5°C
158.7°C
169.0°C
0
50
100
150
200
250
300
Exo Up
Temperature (°C)

FIG. 5

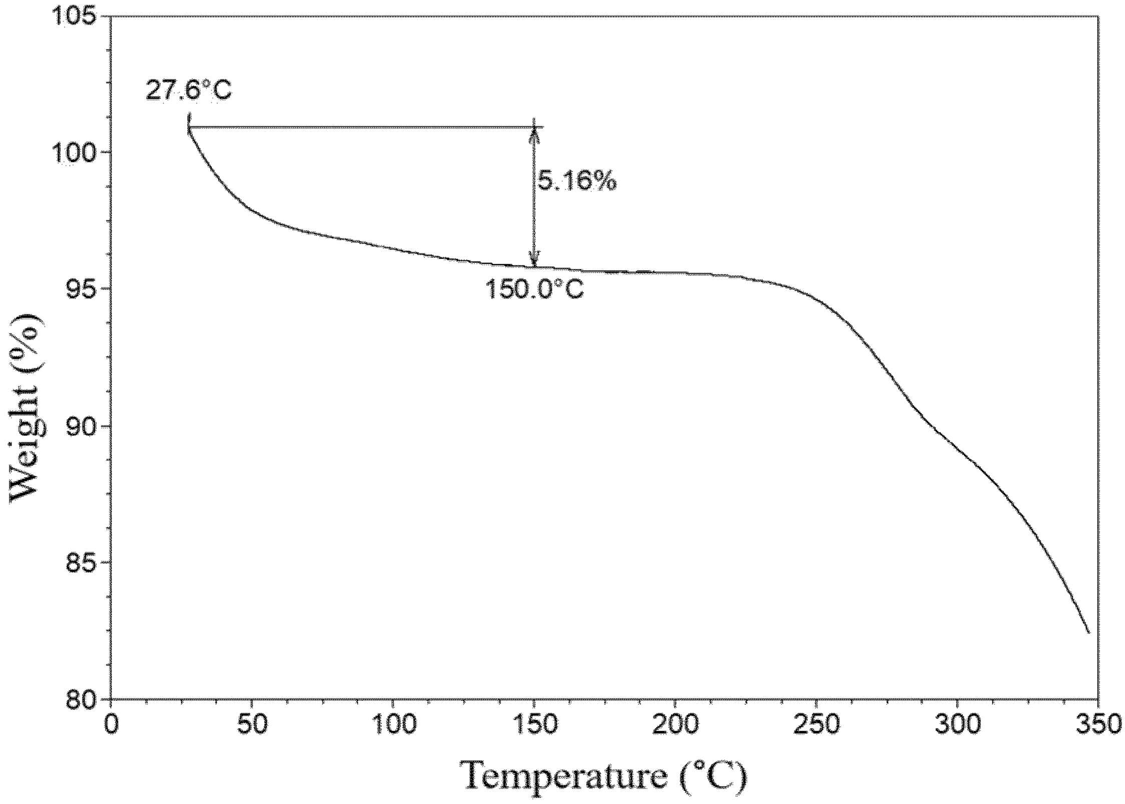
105
100
95
90
85
80
Weight (%)
27.6°C
5.16%
150.0°C
0
50
100
150
200
250
300
350
Temperature (°C)

FIG. 6

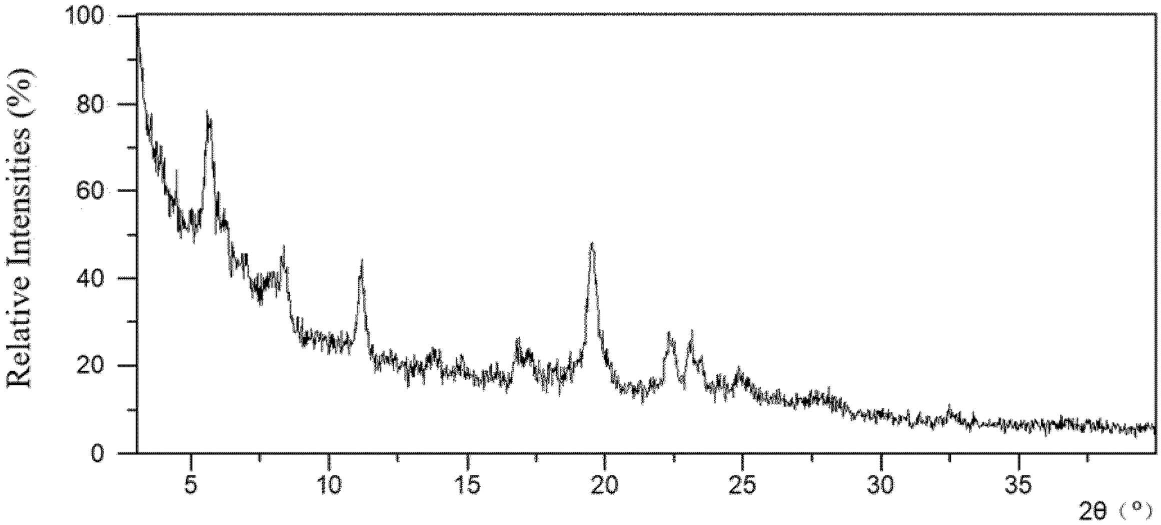
Relative Intensities (%)
100
80
60
40
20
0
5
10
15
20
25
30
35
2θ（°）

FIG. 7

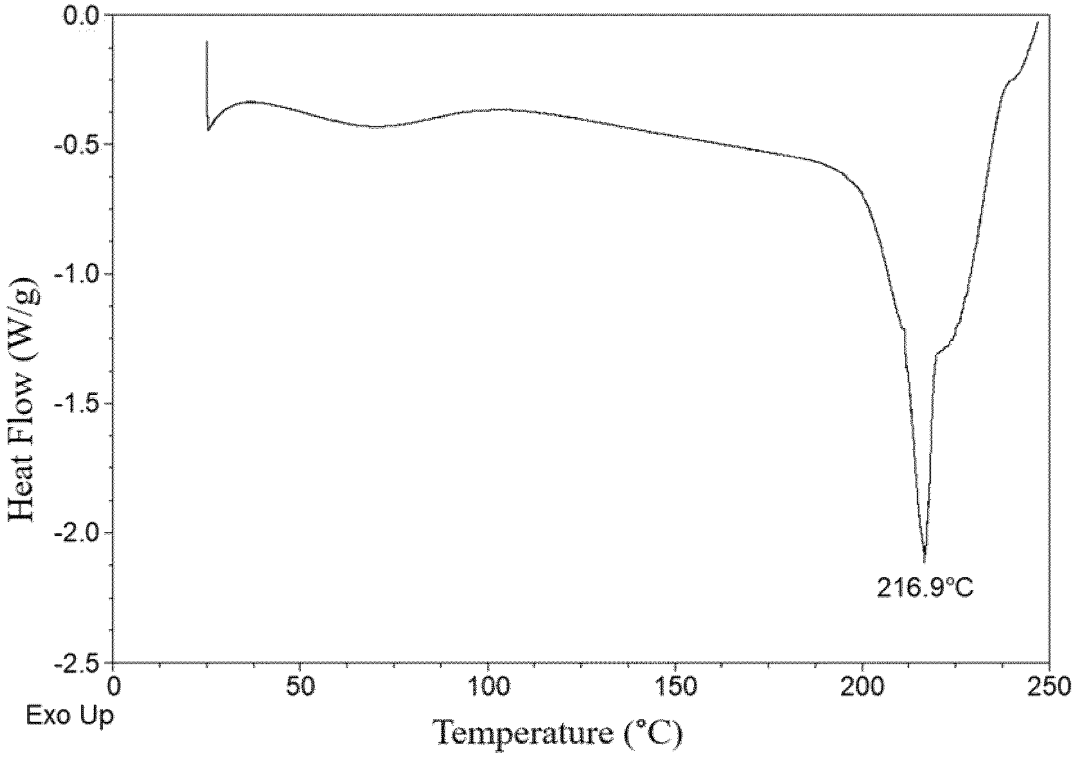
Heat Flow (W/g)
0.0
-0.5
-1.0
-1.5
-2.0
-2.5
216.9°C
Exo Up
0
50
100
150
200
250
Temperature (°C)

FIG. 8

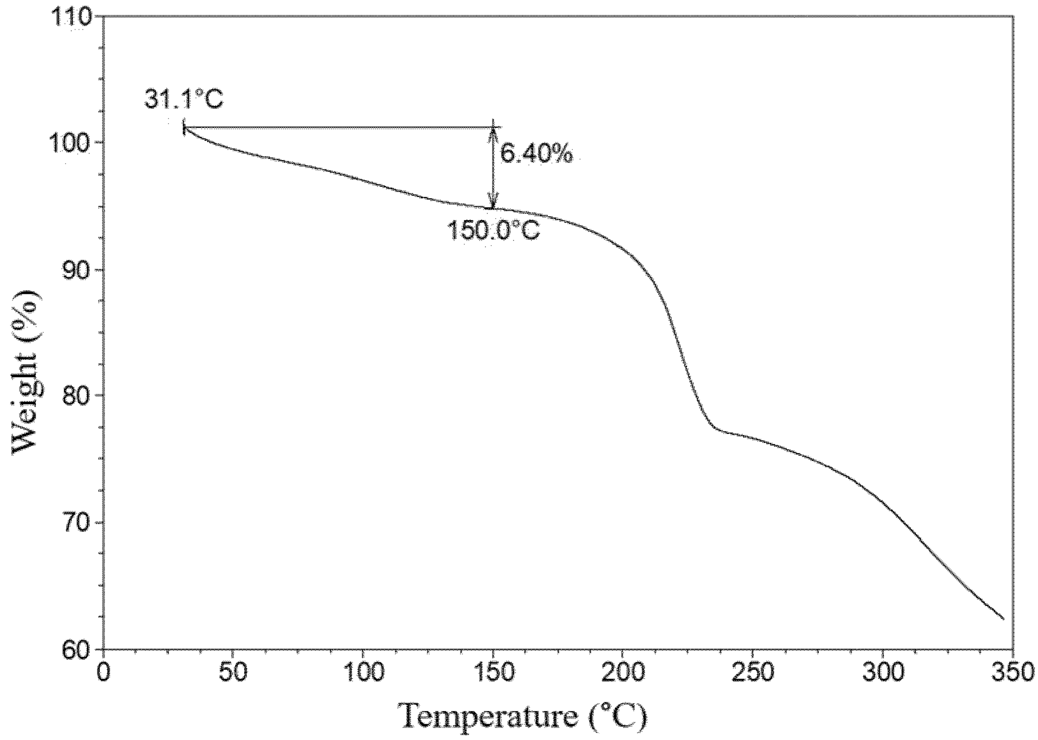
31.1°C
6.40%
150.0°C
110
100
90
80
70
60
Weight (%)
0
50
100
150
200
250
300
350
Temperature (°C)

FIG. 9

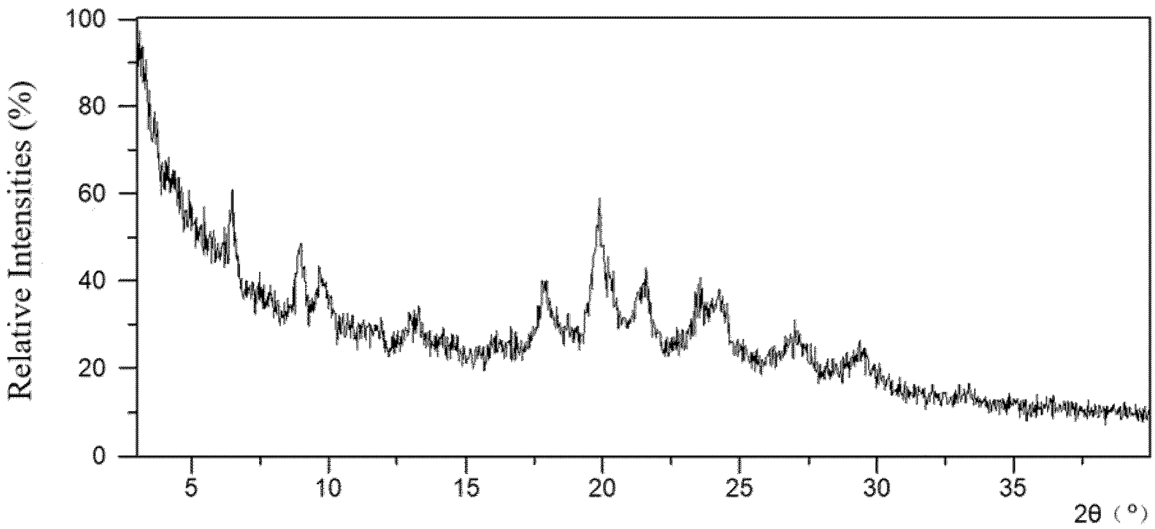
100
80
60
40
20
0
Relative Intensities (%)
5
10
15
20
25
30
35
2θ（°）

FIG. 10

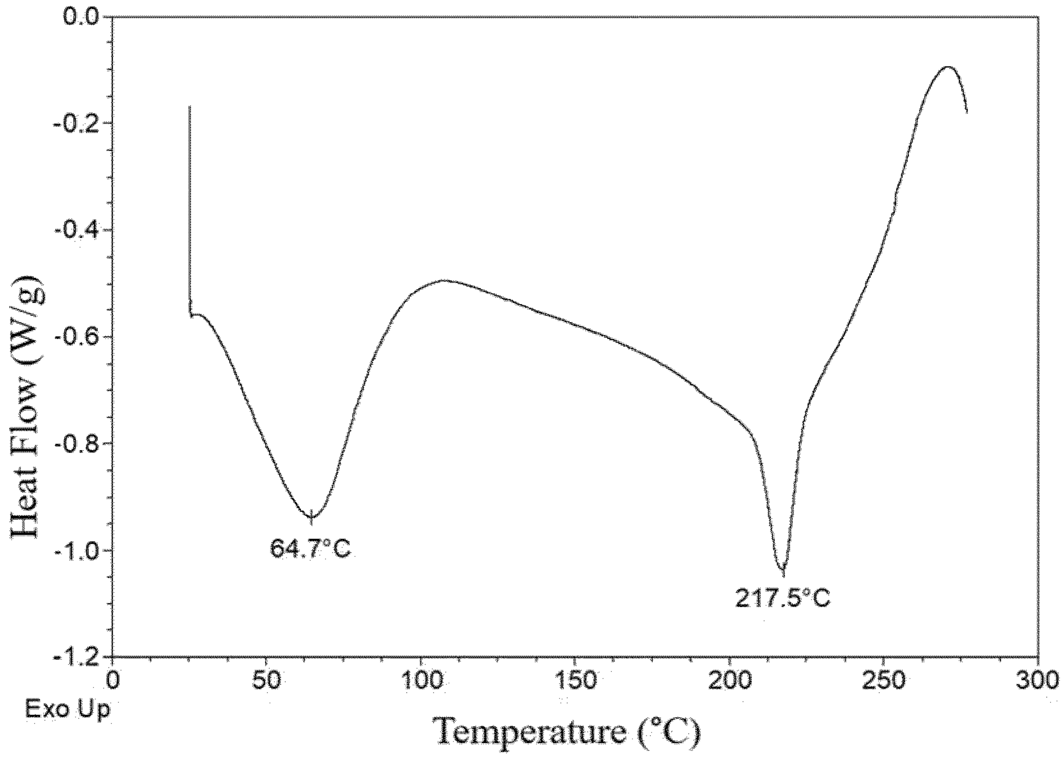
0.0
-0.2
-0.4
-0.6
-0.8
-1.0
-1.2
Heat Flow (W/g)
64.7°C
217.5°C
0
50
100
150
200
250
300
Exo Up
Temperature (°C)

FIG. 11

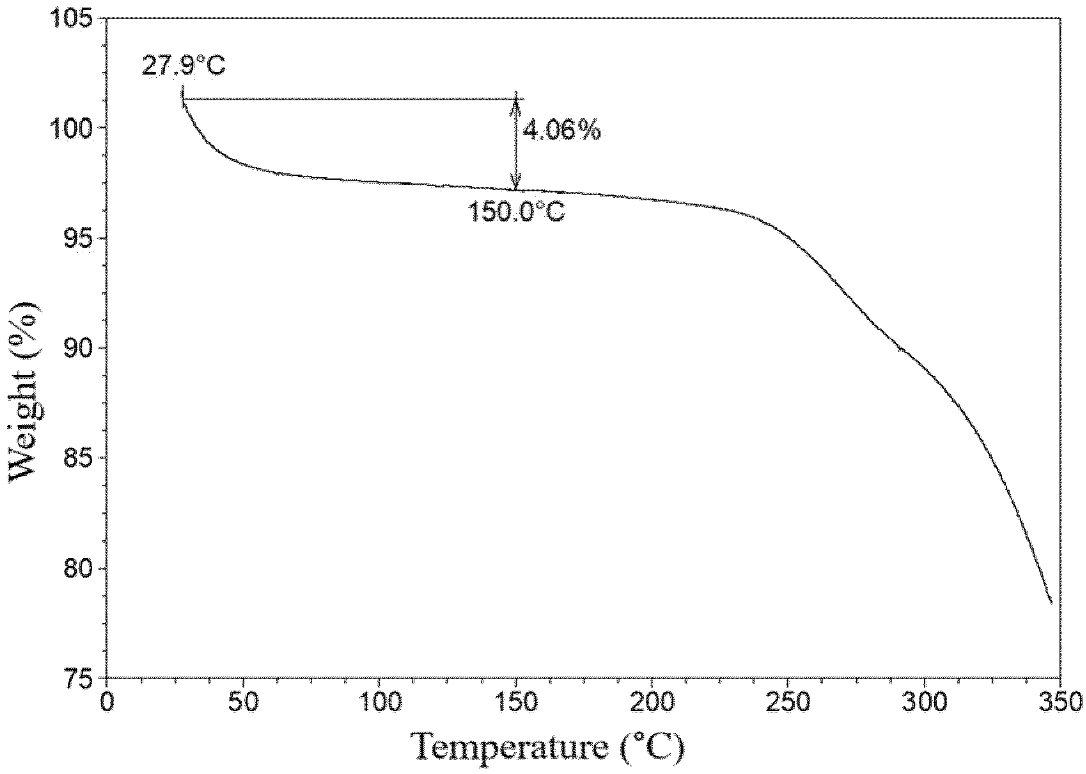
105
100
95
90
85
80
75
Weight (%)
27.9°C
4.06%
150.0°C
0
50
100
150
200
250
300
350
Temperature (°C)

FIG. 12

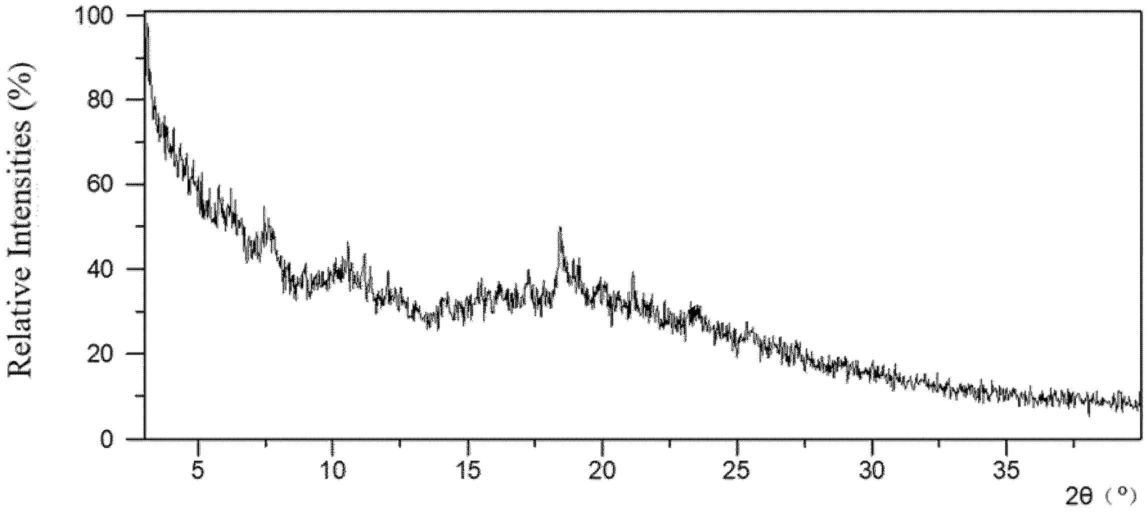
Relative Intensities (%)
100
80
60
40
20
0
5
10
15
20
25
30
35
2θ (°)

FIG. 13

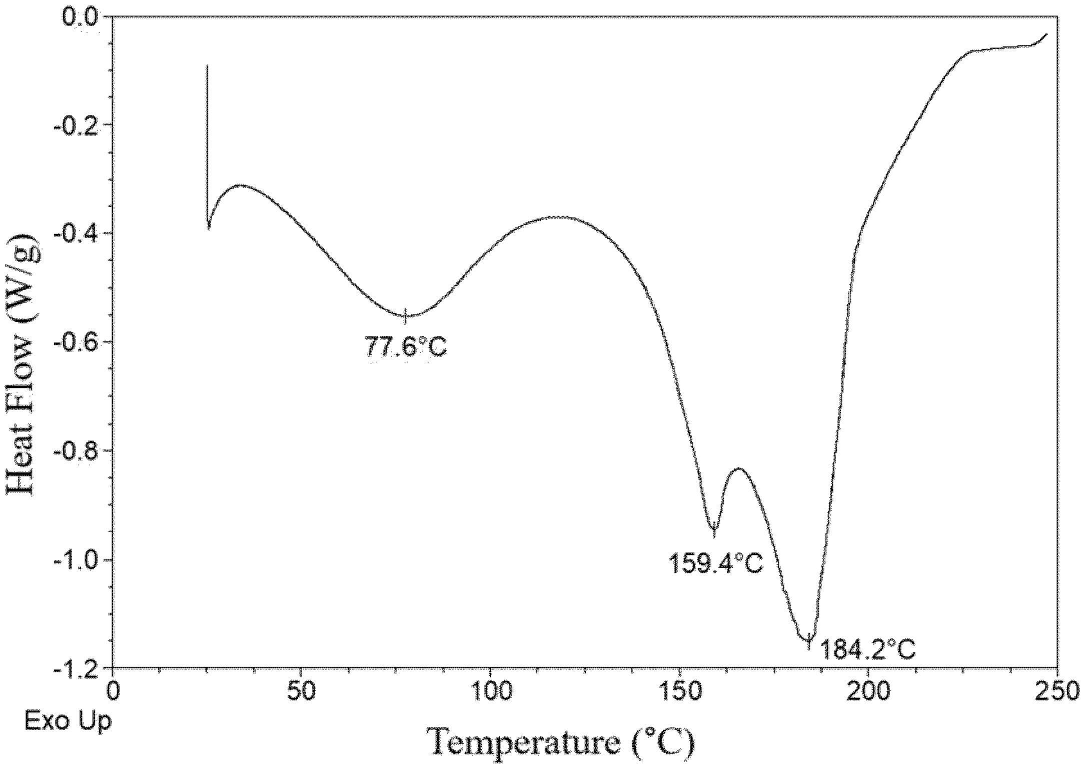
Heat Flow (W/g)
0.0
-0.2
-0.4
-0.6
-0.8
-1.0
-1.2
77.6°C
159.4°C
184.2°C
0
50
100
150
200
250
Exo Up
Temperature (°C)

FIG. 14

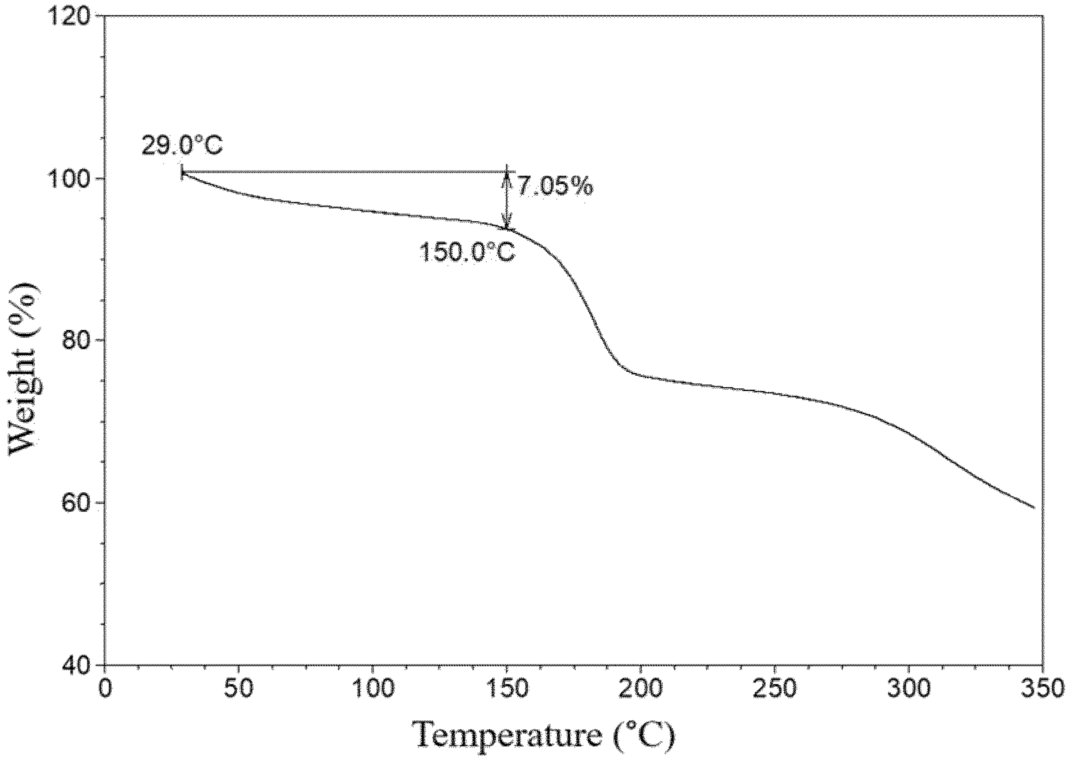


FIG. 15

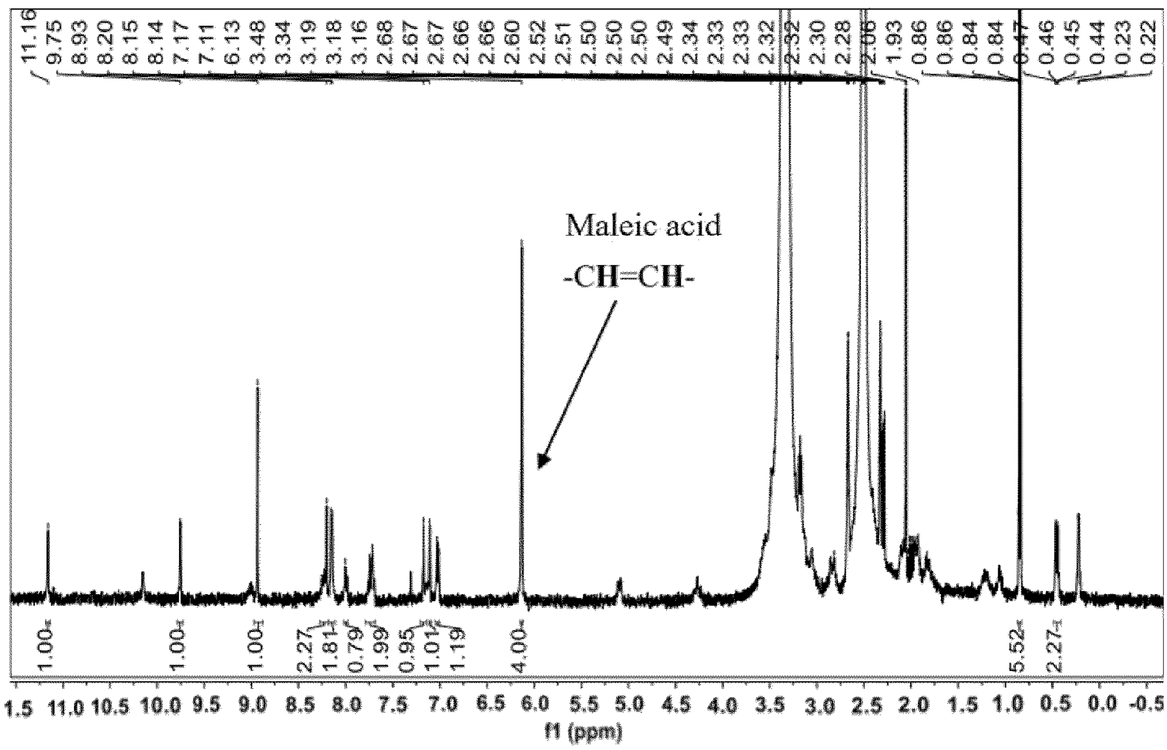


FIG. 16

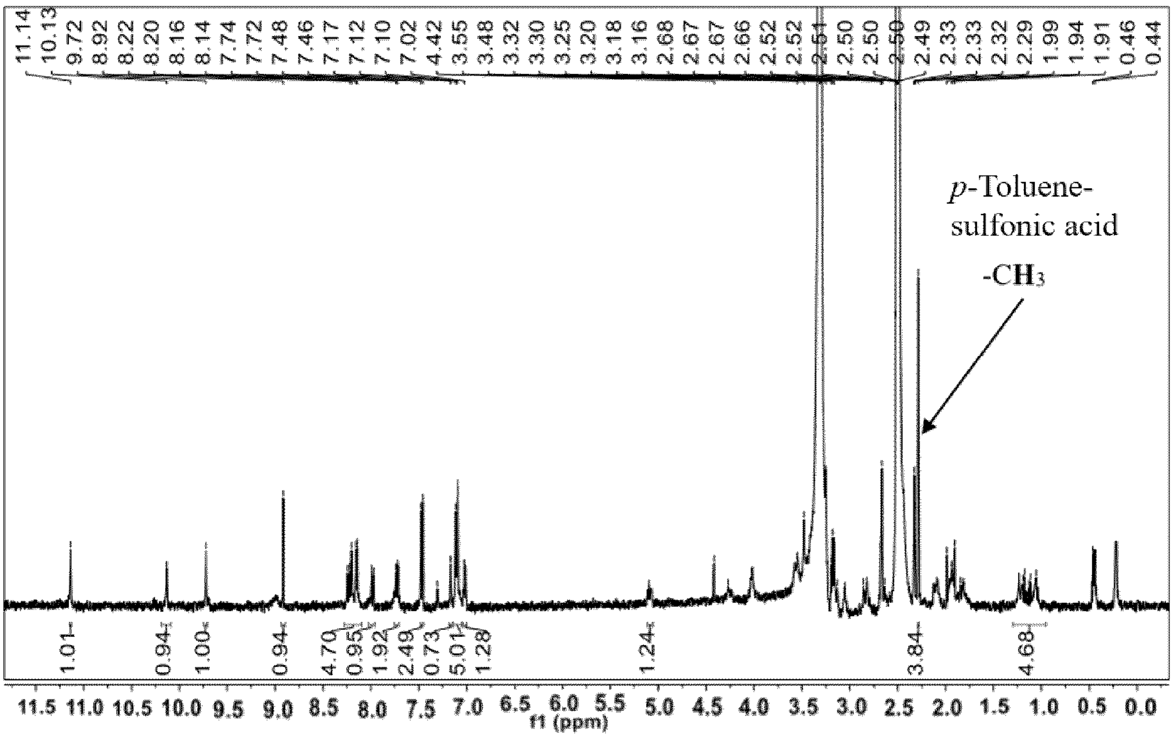
11.14
10.13
9.72
8.92
8.22
8.20
8.16
8.14
7.74
7.72
7.48
7.46
7.17
7.12
7.10
7.02
4.42
3.55
3.48
3.32
3.30
3.25
3.20
3.18
3.16
2.68
2.67
2.67
2.66
2.52
2.52
2.51
2.50
2.50
2.50
2.49
2.33
2.33
2.32
2.29
1.99
1.94
1.91
0.46
0.44
p-Toluene-
sulfonic acid
-CH3
1.01
0.94
1.00
0.94
4.70
0.95
1.92
2.49
0.73
5.01
1.28
1.24
3.84
4.68
11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5 0.0
f1 (ppm)

FIG. 17

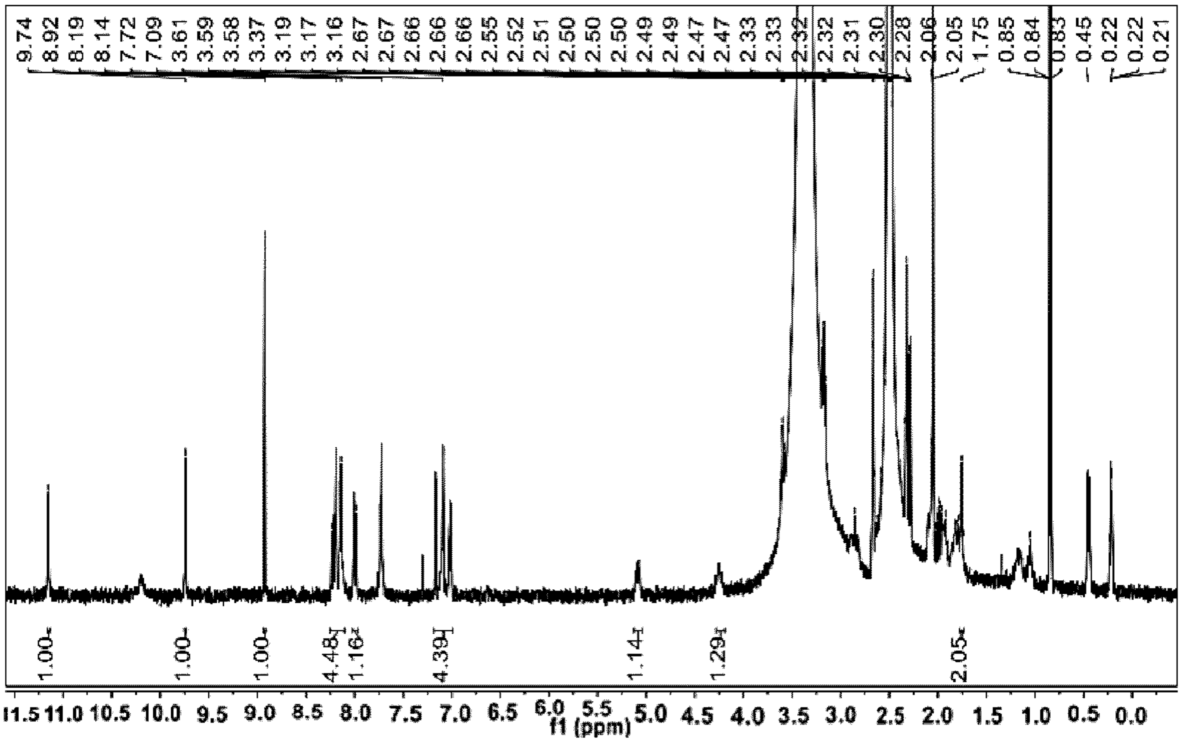


FIG. 18

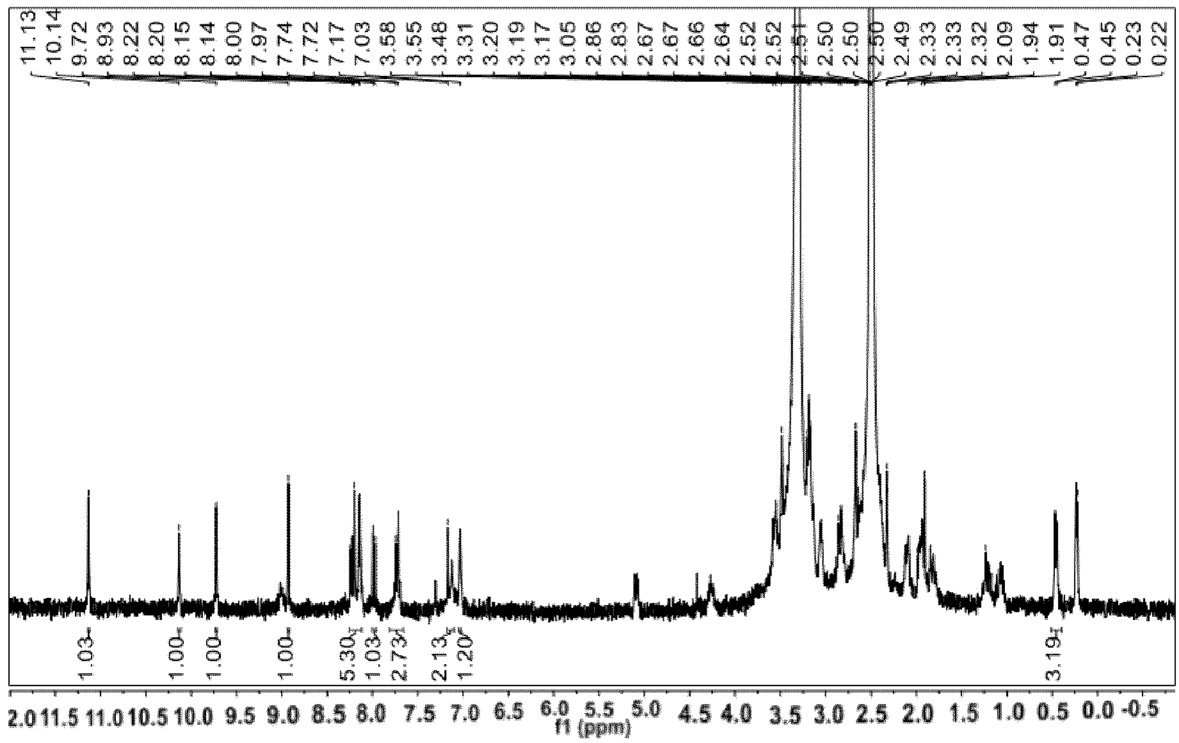


FIG. 19

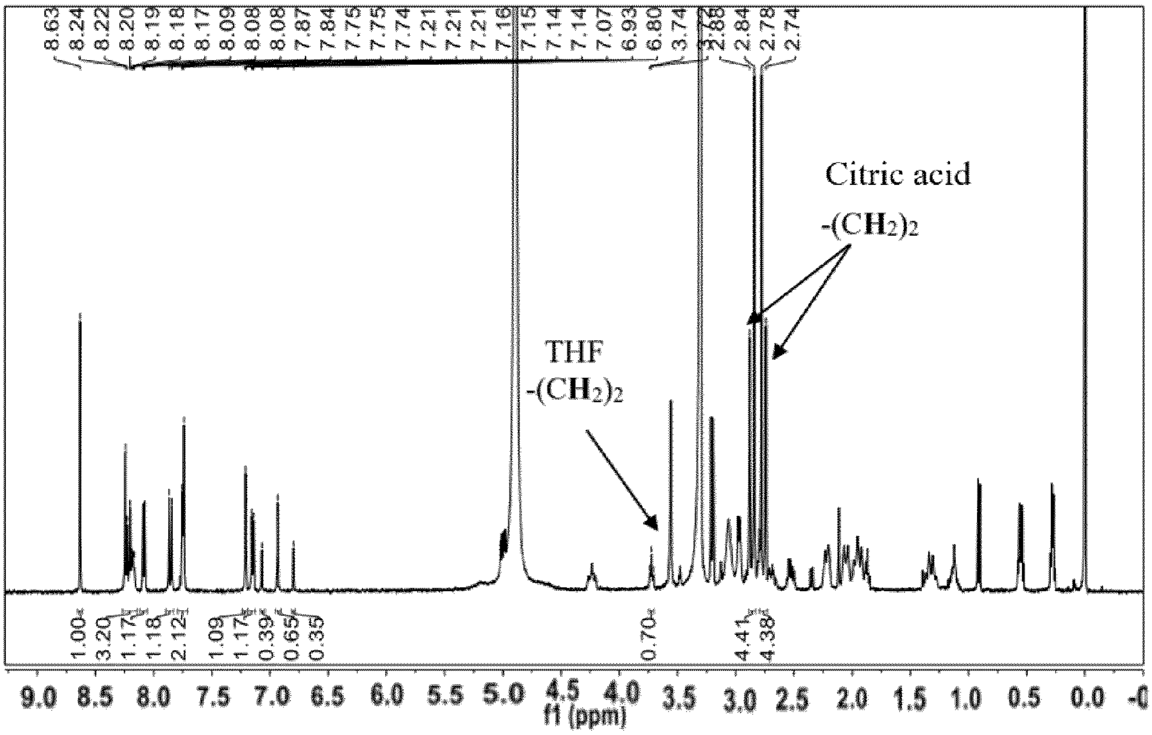
Citric acid
-(CH2)2
THF
-(CH2)2
f1 (ppm)
9.0 8.5 8.0 7.5 7.0 6.5 6.0 5.5 5.0 4.5 4.0 3.5 3.0 2.5 2.0 1.5 1.0 0.5 0.0

FIG. 20

**INTERNATIONAL SEARCH REPORT**

International application No.
**PCT/CN2024/083346**

**A. CLASSIFICATION OF SUBJECT MATTER**

C07D 413/14(2006.01)i; A61K31/4545(2006.01)i; A61K31/496(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D 413/-,A61K,A61P

Documentation searched other than minimum documentation to the extent that such documents are included in the fields searched

Electronic data base consulted during the international search (name of data base and, where practicable, search terms used)

CNKI, CNABS, CNTXT, ENTXT, DWPI, REGISTRY, CAPLUS, MARPAT: 2, 6-哌啶二酮, 白细胞介素, 蛋白降解靶向嵌合体, 肿瘤, 癌症, piperidinedion, IRAK4, PROTAC, CRBN, cancer, tumor, 根据权利要求1进行的structural formula search, structural formula search conducted according to claim 1.

**C. DOCUMENTS CONSIDERED TO BE RELEVANT**

| Category* | Citation of document, with indication, where appropriate, of the relevant passages | Relevant to claim No. |
|---|---|---|
| PX | WO 2023045978 A1 (MEDSHINE DISCOVERY INC.) 30 March 2023 (2023-03-30) entire document | 1-43 |
| A | WO 2020264499 A1 (KYMERA THERAPEUTICS, INC) 30 December 2020 (2020-12-30) entire document, in particular, claims 19-26, and table 1 | 1-43 |
| A | WO 2021127283 A2 (KYMERA THERAPEUTICS, INC.) 24 June 2021 (2021-06-24) entire document, in particular, claims 1, 4 and 25-28, and description, paragraph [0014], and table 1 | 1-43 |
| A | CN 113423427 A (KYMERA THERAPEUTICS, INC.) 21 September 2021 (2021-09-21) entire document, in particular, claims 1, 3 and 20-26, and description, table 1 | 1-43 |
| A | CN 112105385 A (KYMERA THERAPEUTICS, INC.) 18 December 2020 (2020-12-18) entire document | 1-43 |
| A | WO 2021127190 A1 (KYMERA THERAPEUTICS, INC.) 24 June 2021 (2021-06-24) entire document | 1-43 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 July 2024** | **03 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/083346

C. DOCUMENTS CONSIDERED TO BE RELEVANT

| Category* | Citation of document, with indication, where appropriate, of the relevant passages | Relevant to claim No. |
|---|---|---|
| A | WO 2021185291 A1 (MEDSHINE DISCOVERY INC.) 23 September 2021 (2021-09-23) entire document | 1-43 |

Form PCT/ISA/210 (second sheet) (July 2022)

**INTERNATIONAL SEARCH REPORT**
**Information on patent family members**

International application No.
**PCT/CN2024/083346**

| Patent document cited in search report | | | Publication date (day/month/year) | Patent family member(s) | | | Publication date (day/month/year) |
|---|---|---|---|---|---|---|---|
| WO | 2023045978 | A1 | 30 March 2023 | CN | 118055929 | A | 17 May 2024 |
| WO | 2020264499 | A1 | 30 December 2020 | EP | 3989966 | A1 | 04 May 2022 |
| | | | | EP | 3989966 | A4 | 27 September 2023 |
| | | | | JP | 2022538192 | A | 31 August 2022 |
| | | | | US | 2023069104 | A1 | 02 March 2023 |
| | | | | BR | 112021026517 | A2 | 10 May 2022 |
| | | | | CO | 2021017893 | A2 | 17 January 2022 |
| | | | | CA | 3144805 | A1 | 30 December 2020 |
| | | | | KR | 20220032063 | A | 15 March 2022 |
| | | | | IL | 289267 | A | 01 February 2022 |
| | | | | MX | 2021015995 | A | 11 March 2022 |
| | | | | AU | 2020302118 | A1 | 24 February 2022 |
| | | | | CN | 114502158 | A | 13 May 2022 |
| WO | 2021127283 | A2 | 24 June 2021 | US | 2023234953 | A1 | 27 July 2023 |
| | | | | WO | 2021127283 | A3 | 29 July 2021 |
| | | | | US | 2022340570 | A1 | 27 October 2022 |
| | | | | US | 11591332 | B2 | 28 February 2023 |
| | | | | EP | 4076524 | A2 | 26 October 2022 |
| | | | | EP | 4076524 | A4 | 29 November 2023 |
| CN | 113423427 | A | 21 September 2021 | JP | 2022516401 | A | 28 February 2022 |
| | | | | BR | 112021010484 | A2 | 24 August 2021 |
| | | | | CO | 2021007068 | A2 | 30 September 2021 |
| | | | | IL | 283471 | A | 29 July 2021 |
| | | | | CA | 3119773 | A1 | 04 June 2020 |
| | | | | US | 2021323952 | A1 | 21 October 2021 |
| | | | | US | 11352350 | B2 | 07 June 2022 |
| | | | | EP | 3886904 | A1 | 06 October 2021 |
| | | | | EP | 3886904 | A4 | 13 July 2022 |
| | | | | KR | 20210111252 | A | 10 September 2021 |
| | | | | MX | 2021006154 | A | 24 August 2021 |
| | | | | AU | 2019389174 | A1 | 01 July 2021 |
| | | | | US | 2022324854 | A1 | 13 October 2022 |
| | | | | US | 11807636 | B2 | 07 November 2023 |
| | | | | WO | 2020113233 | A1 | 04 June 2020 |
| | | | | US | 11117889 | B1 | 14 September 2021 |
| | | | | SG | 11202105424 | PA | 29 June 2021 |
| CN | 112105385 | A | 18 December 2020 | IL | 304055 | A | 01 August 2023 |
| | | | | AU | 2018396142 | A1 | 16 July 2020 |
| | | | | US | 2023106066 | A1 | 06 April 2023 |
| | | | | US | 11723980 | B2 | 15 August 2023 |
| | | | | JP | 2024038329 | A | 19 March 2024 |
| | | | | SG | 11202005912 | PA | 29 July 2020 |
| | | | | US | 2019192668 | A1 | 27 June 2019 |
| | | | | US | 10874743 | B2 | 29 December 2020 |
| | | | | BR | 112020012997 | A2 | 01 December 2020 |
| | | | | MX | 2020006812 | A | 06 November 2020 |
| | | | | CA | 3086763 | A1 | 04 July 2019 |
| | | | | JP | 2021508703 | A | 11 March 2021 |
| | | | | EP | 3731869 | A1 | 04 November 2020 |
| | | | | EP | 3731869 | A4 | 22 September 2021 |

Form PCT/ISA/210 (patent family annex) (July 2022)

**INTERNATIONAL SEARCH REPORT**
**Information on patent family members**

International application No.
**PCT/CN2024/083346**

| Patent document cited in search report | Publication date (day/month/year) | Patent family member(s) | Publication date (day/month/year) |
|---|---|---|---|
| | | US 2023398223 A1 | 14 December 2023 |
| | | WO 2019133531 A1 | 04 July 2019 |
| | | IL 275649 A | 31 August 2020 |
| | | IL 275649 B1 | 01 August 2023 |
| | | IL 275649 B2 | 01 December 2023 |
| | | US 11318205 B1 | 03 May 2022 |
| WO 2021127190 A1 | 24 June 2021 | BR 112022011651 A2 | 23 August 2022 |
| | | US 2024131016 A1 | 25 April 2024 |
| | | MX 2022007576 A | 23 September 2022 |
| | | AU 2020407200 A1 | 21 July 2022 |
| | | EP 4076520 A1 | 26 October 2022 |
| | | EP 4076520 A4 | 27 March 2024 |
| | | CO 2022008406 A2 | 08 July 2022 |
| | | CA 3161878 A1 | 24 June 2021 |
| | | TW 202136251 A | 01 October 2021 |
| | | IL 293917 A | 01 August 2022 |
| | | KR 20220145325 A | 28 October 2022 |
| | | JP 2023509366 A | 08 March 2023 |
| | | US 2023144292 A1 | 11 May 2023 |
| | | US 11707457 B2 | 25 July 2023 |
| | | US 2021228562 A1 | 29 July 2021 |
| | | US 11779578 B2 | 10 October 2023 |
| | | CN 115052627 A | 13 September 2022 |
| WO 2021185291 A1 | 23 September 2021 | US 2023158152 A1 | 25 May 2023 |
| | | EP 4122925 A1 | 25 January 2023 |
| | | EP 4122925 A4 | 17 April 2024 |
| | | JP 2023517393 A | 25 April 2023 |
| | | CN 115380026 B | 22 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023102949509